# EUROPEAN PATENT APPLICATION

(11) **EP 4 495 239 A1**
(43) Date of publication of application: **22.01.2025**
(21) Application number: 23186682.3
(22) Date of filing: 20.07.2023
(51) Int. Cl.: C12N 15/113, A61K 31/713

(54) **AGENTS FOR TREATING CANCER**

(71) Applicant: Toumpoulis, Ioannis, 25200 Kato Achaia (GR)
(72) Inventor: Toumpoulis, Ioannis, 25200 Kato Achaia (GR)
(74) Representative: ZBM Patents - Zea, Barlocci & Markvardsen

(57) **Abstract**

The present disclosure provides a combination of oligonucleotides, wherein said combination reduces the expression of the genes COL5A2, COL11A1, MMP9, ITGB1, TGFB1 and BMP1 and use thereof in treating cancer, in particular, metastatic cancer.

## Description

### Technical Field

The present disclosure pertains to the field of medical treatment, in particular it refers to the treatment of cancer by gene therapy.

### Background Art

Cancer ranks as a leading cause of mortality and represents an important disease resulting in decreased life expectancy worldwide. According to estimates from the World Health Organization in 2019, cancer is the first or second leading cause of death before the age of 70 years in 112 of 183 countries including the geographic areas of North and South America, Europe, China and Australia. Worldwide, an estimated 28.4 million new cancer cases are projected to occur in 2040, which represents a 47% increase from the corresponding 19.3 million new cases in 2020. Given the fact that in 2020 there were reported approximately 10 million cancer-related deaths, it is obvious that such a dramatic increase in cancer cases in the coming decades will likely be paralleled with increased mortality rates, unless effective therapeutic interventions are developed.

It is important to consider that the worse progression of cancer toward advanced pathological stages implies invasion and metastasis processes. Indeed, in the presence of distant metastases the prognosis is very poor. The metastatic disease complicates the treatment of patients and it is the cause of almost 90% of cancer deaths.

To date there are no effective therapeutic interventions to prevent the ability of common primary tumors to surmount physical boundaries, disseminate and finally colonize a distant organ. There is therefore an urgent need to develop novel therapeutic interventions specifically designed to prevent, slow down or treat metastasis.

### Summary of Invention

The inventor has surprisingly found that it is possible to reduce or attenuate tumor cell migration by gene therapy targeting particular genes that code for extracellular matrix (ECM) structural proteins, enzymes and transmembrane receptors mediating cell-ECM interaction, said genes including COL5A2, COL11A1, matrix metalloproteinases (MMPs), Integrin subunits, TGFB1 and BMP1.

Thus, a first aspect of the present disclosure provides for a combination of oligonucleotides, wherein said combination reduces or supresses the expression of one or more genes selected from the group consisting of COL5A2, COL11A1, MMP9, ITGB1, TGFB1 and BMP1.

Without wanting to be bound by theory, the inventor hypothesizes that down-regulation of the above-mentioned genes in cancer patients leads to increased gene and protein expression levels of integral components of the ECM. This impaired microRNA (miRNA) regulatory mechanism of gene expression in turn leads to a remodelling of ECM which favours development and progression of cancer. The inventor has shown that gene therapy intervention may interfere with this pathogenic mechanism and reduce tumor cell migration, resulting in a novel therapeutic strategy for controlling invasion and metastasis in cancer.

As shown in the examples below, the inventor has designed gene-specific, synthetic, small interfering RNA (siRNA) oligonucleotides that induce gene silencing of COL5A2, COL11A1, MMP9, ITGB1, TGFB1 and BMP1 and reduce cell migration in osteosarcoma U-2 OS and lung adenocarcinoma A549 cell lines, thus confirming the anti-cancer anti-metastatic effect of the herein disclosed gene therapy intervention.

The combination of oligonucleotides disclosed herein may be provided as a composition, for example, a pharmaceutical composition ready for administration to a subject in need thereof. Thus, a second aspect of the invention refers to a composition, for example, a pharmaceutical composition, comprising a combination of oligonucleotides according to the first aspect.

A third aspect of the present disclosure is directed to a combination of oligonucleotides as defined in the first aspect, or a composition as defined in the second aspect, for use as a medicament. This can be rephrased as the use of combination of oligonucleotides as defined in the first aspect, or a composition as defined in the second aspect, for the preparation of a medicament. Also disclosed is a method for treating a subject in need thereof, the method comprising administering a combination of oligonucleotides as defined in the first aspect or a composition as defined in the second aspect.

A fourth aspect of the present disclosure is directed to a combination of oligonucleotides as defined in the first aspect, or a composition comprising said combination of oligonucleotides as defined in the second aspect, for use in treating cancer. This can be rephrased as the use of combination of oligonucleotides as defined in the first aspect, or a composition as defined in the second aspect, for the preparation of a medicament for treating cancer. Also disclosed is a method for treating cancer in a subject in need thereof, the method comprising administering a combination of oligonucleotides as defined in the first aspect or a composition comprising said combination of oligonucleotides as defined in the second aspect.

The present inventor also surprisingly found that administration of heparin or a heparin surrogate, including antibodies that bind to the heparin binding domain within the proteins encoded by the above-mentioned genes, further enhances the anti-metastatic effect of the oligonucleotide combination of the first aspect. It is though that the combination of heparin, or heparin surrogate, and the combination of oligonucleotides of the first aspect act synergically in blocking invasiveness in cancer by reducing the level of expression of the targeted genes at the molecular level and inhibiting abnormal protein interactions at the protein level.

Interestingly, the inventor has also found several heparin-binding domains withing each of the ECM-related proteins of interest. Such an example of heparin-binding domain can be expressed as XBBBXXBX, where B designates a basic residue and X indicates any other residue. Stemming from this finding, the inventor has designed antibodies that specifically bind to this heparin-binding domain, thus effectively mimicking the effect obtained by heparin in blocking abnormal interactions of the ECM proteins.

The present disclosure thus provides, in a fifth aspect, a protein inhibitor that binds to a heparin-binding domain within a protein selected from COL5A2, COL11A1, MMP9, ITGB1, TGFB1 and BMP1.The protein inhibitor can be an antibody or any other compound which specifically binds to a heparin-binding domain within a protein selected from COL5A2, COL11A1, MMP9, ITGB1, TGFB1 and BMP1. The heparin-binding domain for each of the proteins of interest is shown in tables 3 to 11. Moreover, in a sixth aspect the disclosure provides a combination of protein inhibitors defined in the fifth aspect.

In a sixth aspect, the present disclosure refers to a combination of oligonucleotides as defined in the first aspect or a composition defined in the second aspect for use in treating cancer, when used in combined therapy with heparin, a heparin surrogate, or one or more protein inhibitors, as defined in the fifth aspect. This aspect can also be formulated as the use of a combination of oligonucleotides as defined in the first aspect or a composition as defined in the second aspect for the manufacture of a medicament for treating cancer in combined therapy with heparin, a heparin surrogate, or one or more protein inhibitors as defined in the fifth aspect. This aspect can also be formulated as a method for treating cancer, the method comprising administering to a subject in need thereof a combination of oligonucleotides as defined in the first aspect or a composition as defined in the second aspect and heparin, a heparin surrogate, or one or more protein inhibitors, as defined in the fifth aspect.

A seventh aspect refers to heparin, a heparin surrogate, or a one or more protein inhibitors as defined in the fifth aspect, for use in treating cancer, when used in combined therapy with a combination of oligonucleotides as defined in the first aspect or a composition defined in the second aspect. This aspect can also be formulated as the use of heparin, a heparin surrogate, or one or more protein inhibitors as defined in the fifth aspect, for the manufacture of a medicament for treating cancer in combined therapy with a combination of oligonucleotides as defined in the first aspect or a composition as defined in the second aspect.

While reducing or suppressing cancer metastasis, the herein disclosed therapeutic approach is particularly suitable for combined therapy for cancer treatment. Combining two or more therapeutic strategies is a cornerstone of cancer therapy. In this sense, anti-metastatic therapeutic approaches are of great interest to stop cancer progression, since, as outlined above, metastatic disease complicates the treatment of patients and is the cause of almost 90% of cancer deaths.

The present disclosure thus further refers, in another aspect, to a combination of oligonucleotides as defined in the first aspect or a composition as defined in the second aspect for use in treating cancer, when used in combined therapy with surgery, radiation or an antitumoral agent for the treatment of cancer. This aspect can also be formulated as the use of a combination of oligonucleotides as defined in the first aspect or a composition as defined in the second aspect for the manufacture of a medicament for the treatment of cancer in combined therapy with surgery, radiation or an antitumoral agent. This aspect can also be formulated as a method for treating cancer, the method comprising administering to a subject in need thereof a combination of oligonucleotides as defined in the first aspect or a composition as defined in the second aspect and surgery, radiation or an antitumoral agent.

Finally, the present disclosure provides for a kit of parts comprising: (a) a combination of oligonucleotides as defined in the first aspect, optionally (b) heparin, a heparin surrogate, or one or more protein inhibitors as defined in the fifth aspect, and, optionally, (c) instructions for its use in the treatment of cancer.

### Brief Description of Drawings

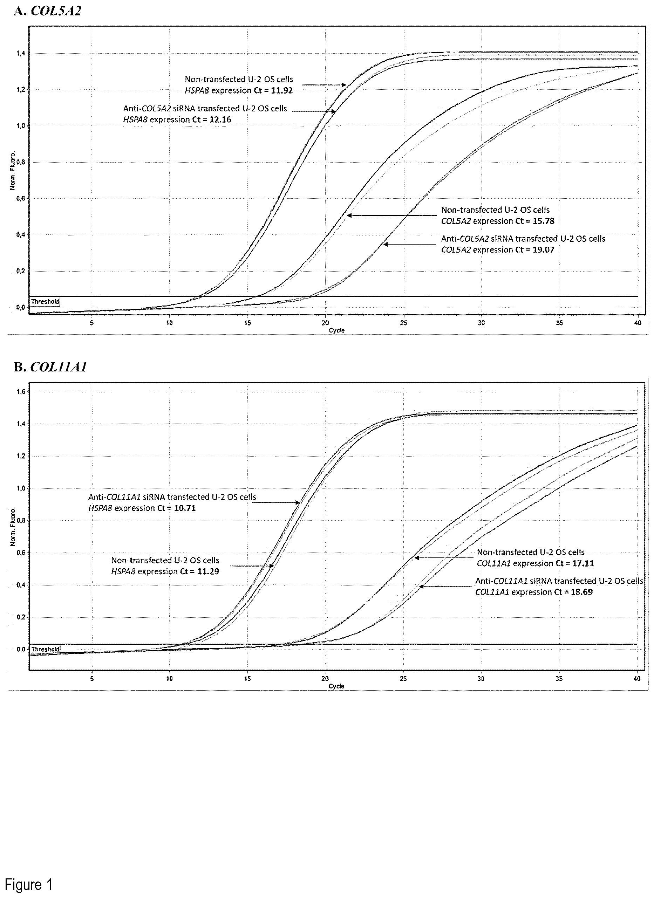

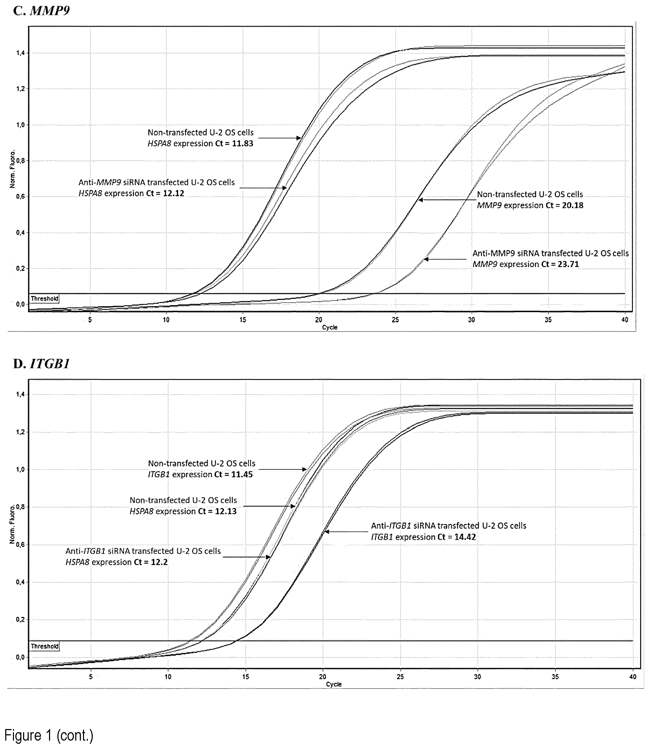

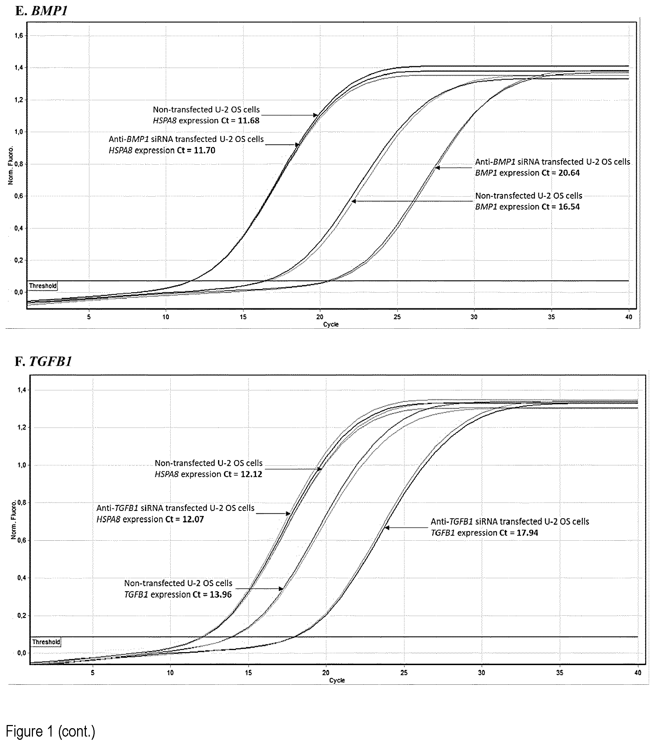

Figure 1: mRNA levels of COL5A2, COL11A1, MMP9, ITGB1, BMP1 and TGFB1 in non-transfected and siRNA-transfected U-2 OS cells were analyzed by RT-qPCR. A-F: Amplification curves and Ct values are given for each target-gene, indicatively. HSPA8 expression levels were also determined both in non-transfected and transfected cells.

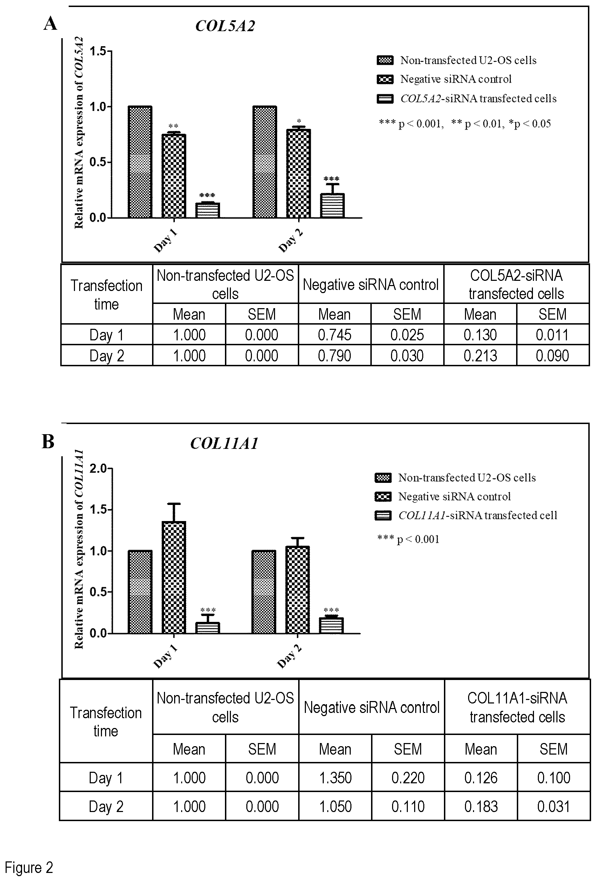

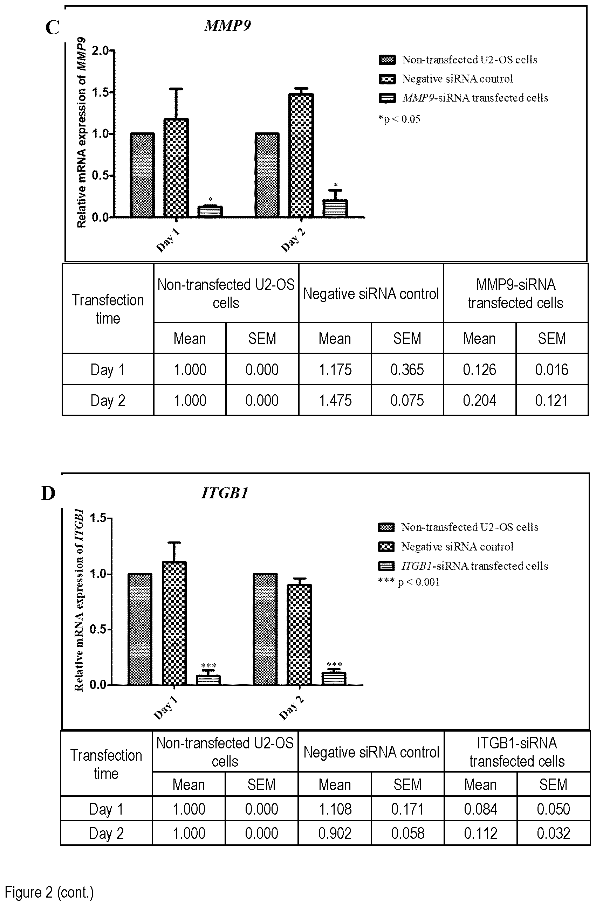

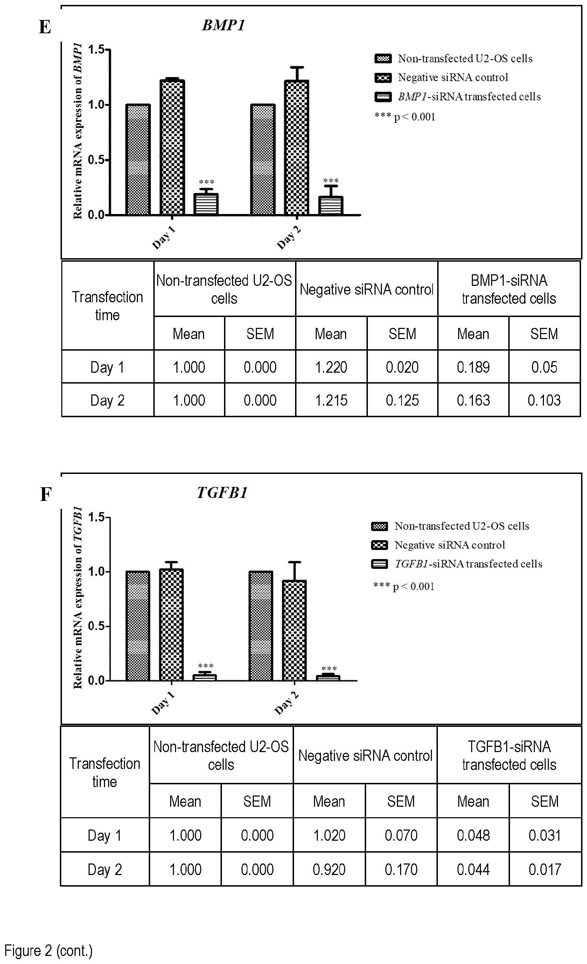

Figure 2: siRNA-mediated silencing efficiency of COL5A2, COL11A1, MMP9, ITGB1, BMP1 and TGFB1 in U-2 OS cells. (A-F) Relative mRNA expression of target-genes was determined by RT-qPCR 1- and 2-days post-transfection, using non-transfected U-2 OS cells as the reference sample. Data are presented as the mean ± SEM of 2 independent experiments. Statistical analysis was performed using Two-way ANOVA with Bonferroni post-hoc analysis. Statistical differences are indicated by *p < 0.05, **p < 0.01 and ***p < 0.001.

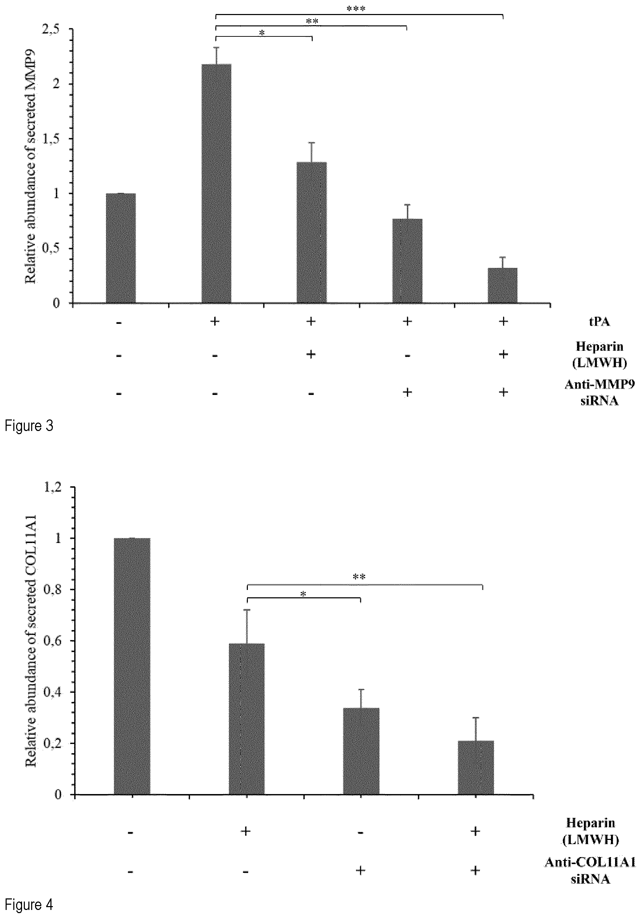

Figure 3: Graphical presentation of the relative abundance of secreted MMP9 in the absence or presence of low molecular weight heparin (LMWH) and/or anti-MMP9 siRNA, in cells previously stimulated with tPA Heparin or anti-MMP9 siRNA administration, separately, causes significant reduction of secreted MMP9 levels in U-2 OS cells pre-treated with tPA. Co-treatment of cells with both heparin and anti-MMP9 siRNA further inhibited the secretion of MMP9. Starved cells without further treatment/stimulation served as negative control. Data are presented as the mean ± SEM of 2 independent experiments. Statistical analysis was performed using One-way ANOVA (*p<0.05, **p<0.01, ***p<0.001, N=2).
Figure 4: Graphical presentation of the relative abundance of secreted COL11A1 in U-2 OS cells treated with LMWH or anti-COL11A1 siRNA, separately and combined. Co-treatment of cells with heparin and anti-COL11A1 siRNA suppressed even more the secretion of COL11A1. Starved cells without further treatment/stimulation served as negative control. Data are presented as the mean ± SEM of 2 independent experiments. Statistical analysis was performed using One-way ANOVA (*p<0.05, **p<0.01, N=2).

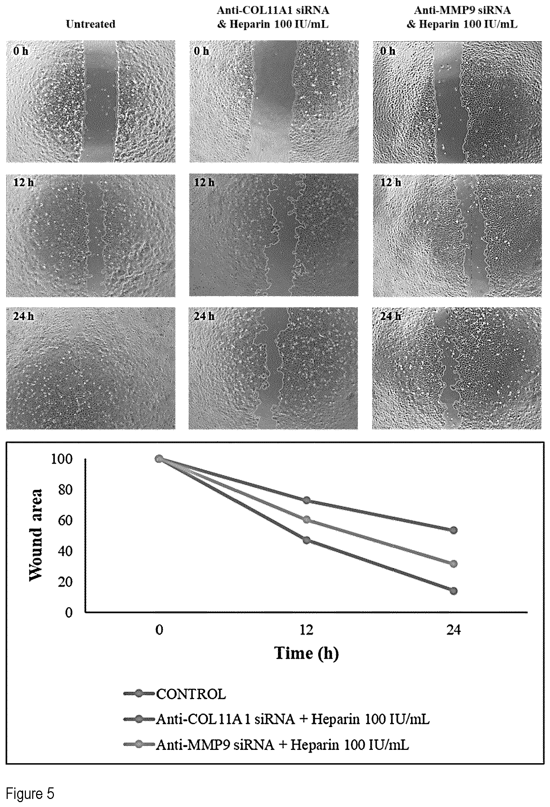

Figure 5: Analysis of U-2 OS cells migration using in vitro wound healing assay. Above: microscopy images of wound closure in monolayer of untreated vs. treated cells at 0, 12 & 24 h after scratch formation. Below: Relative quantification of the wound area invaded during 24 h by untreated and treated cells.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

A "cancer", as used herein, is any unwanted growth of cells serving no physiological function. In general, a cancer cell has been released from its normal cell division control, i.e., a cell whose growth is not regulated by the ordinary biochemical and physical influences in the cellular environment. Thus, "cancer" is a general term for diseases characterized by abnormal uncontrolled cell growth. In most cases, a cancer cell proliferates to form clonal cells that are either benign or malignant. The resulting lump or cell mass, or "tumor," is generally capable of invading and destroying surrounding normal tissues. By "malignant tumor" is meant an abnormal growth of any cell type or tissue, that has a deleterious effect in the organism having the abnormal growth. The term "malignancy" or "cancer" includes cell growths that are technically benign but which carry the risk of becoming malignant. Tumoral cells may spread from their original site to other parts of the body through the lymphatic system or blood stream in a process known as "metastasis" causing a "secondary tumor" that results in "metastatic cancer" disease.

### Oligonucleotides

The present disclosure provides oligonucleotides that are able to downregulate the expression of COL5A2, COL11A1, MMP9, ITGB1, TGFB1 and/or BMP1. The complete name and NCBI reference for the genes of interest is provided in table 1.

**Table 1**

| Gene name | Symbol | NCBI Reference Sequence |
|---|---|---|
| collagen type XI alpha 1 chain | *COL11A1* | NG_008033.1 |
| collagen type V alpha 2 chain | *COL5A2* | NG_011799.2 |
| transforming growth factor beta-1 | *TGFB1* | NG_013364.1 |
| integrin subunit beta 1 | *ITGB1* | NG_029012.1 |
| matrix metallopeptidase 9 | *MMP9* | NG_011468.1 |
| bone morphogenetic protein 1 | *BMP1* | NG_029659.1 |

By "downregulating" the expression of a gene it is meant that the expression is reduced, such that the encoded protein is produced in lower levels. In particular embodiments, expression of the encoded protein(s) is reduced by 5%, 10%, 15%, 20%, 25%, 30%, 35%, 40%, 45%, 50%, 60%, 70%, 80%, 90% or 100%. Hence, by employing the oligonucleotides of the present disclosure, the pathogenic effect of an overexpression of said ECM-integrating proteins is ameliorated and the migration and invasiveness of cancer is reduced. In the present disclosure the expressions "dowregulating the expression" and "reducing the expression" are used interchangeably.

Reduction of the expression of the encoded protein(s) can be measured by any suitable technique known in the art. For example, reverse transcription, Sanger sequencing and/or quantitative real-time PCR are frequently used techniques. The examples below describe a method to determine the expression of the genes in greater detail.

In the context of this disclosure, the term "oligonucleotide" refers to an oligomer of nucleotide or nucleoside monomers consisting of naturally-occurring bases, sugars and intersugar (backbone) linkages. Oligonucleotides are generally classified as deoxyribooligonucleotides or ribooligonucleotides, which are oligomers of DNA or RNA molecules. A deoxyribooligonucleotide consists of a 5-carbon sugar (deoxyribose) which is joined covalently to phosphate at the 5' and 3' carbons of the sugar to form an alternating, unbranched polymer. A ribooligonucleotide consists of a similar repeating structure where the 5-carbon sugar is ribose.

The term "oligonucleotide" also includes oligomers comprising non-naturally occurring monomers, or portions thereof, which function similarly. Such modified or substituted oligonucleotides are sometimes preferred over native forms because of properties such as, for example, enhanced cellular uptake, reduced immunogenicity, and increased stability in the presence of nucleases. The following paragraphs describe non-limiting modifications to the oligonucleotides.

In some embodiments, the oligonucleotides contain a phosphorothioated backbone. For example, none, 5 or more, 10 or more, 15 or more, 20 or more, 25 or more, or all of the oligonucleotide bases have a phosphorothioated backbone. Oligonucleotides that contain phosphorothioated backbones provide an increased resistance to nucleases compared to unmodified oligonucleotides (containing 100% phosphodiester backbone). In some embodiments, the oligonucleotides comprise modifications to help enhance their properties. Hence, in some embodiments the oligonucleotide may be modified by the substitution of at least one nucleotide with at least one modified nucleotide, ideally so that the in vivo and in vitro stability of the oligonucleotide is enhanced as compared to a corresponding unmodified oligonucleotide. In some embodiments, the oligonucleotides comprise 2'-deoxy guanosine, 2'-deoxy adenosine, 2'-0-methylguanosine, 2'-0-methyl (e.g., 2'- O-methylcytidine, 2'-0-methylpseudouridine, 2'-0-methyluridine, 2'-0-methyladenosine, 2'-0-methyl) ribonucleotide, 2'- amino, 2'-thio and 2'-fluoro modified ribonucleotide, 2'-fluoro-cytidine, 2'-fluoro-uridine, 2'- fluoro-guanosine, 2'-fluoro-adenosine, 2'-amino-cytidine, 2'-amino-uridine, 2'-amino- adenosine, 2'-amino-guanosine, 2'-amino-butyryl-pyrene-uridine, 2'-amino-adenosine, 5-iodo-uridine, ribo- thymidine, 5-bromo-uridine, 2-aminopurine, 5-methyl-cytidine , 5-fluoro-cytidine, and 5- fluoro-uridine, 2,6-diaminopurine, 4-thio-uridine, and/or 5-amino-allyl-uridine.

In some embodiments, the oligonucleotides include derivatization of the 5 position, for instance being selected from 5-(2-amino) propyl uridine, 5-bromo uridine, 5-propyne uridine, 5-propenyl uridine; derivatization of the 6 position, for instance 6-(2-amino)propyl uridine; derivatization of the 8-position for adenosine and/or guanosines, for instance 8- bromo guanosine, 8-chloro guanosine, or 8-fluoroguanosine. In other embodiments, the oligonucleotides comprise nucleotide analogs such as deaza nucleotides, e.g., 7-deaza-adenosine; O- and N-modified (for instance alkylated, such as N6-methyl adenosine) nucleotides; and other heterocyclically modified nucleotide analogs.

In other embodiments, the oligonucleotides comprise a modified sugar portion. Examples of modifications to the sugar portion of the nucleotides which may be employed include the 2' OH-group being replaced by a group selected from H, OR, R, F, Cl, Br, I, SH, SR, H2, NHR, NR2, COOR, or OR, wherein R is substituted or unsubstituted C1-C6 alkyl, alkenyl, alkynyl, aryl and so on. The phosphate group of the nucleotide may also be modified, such as by substituting one or more of the oxygens of the phosphate group with sulphur (for instance by employing phosphorothioates). Modifications may decrease the rate of hydrolysis of polynucleotides comprising the modified bases, for example by inhibiting degradation by exonucleases. In one preferred instance, the oligonucleotide is resistant to ribonucleases. Oligonucleotides which may be employed include those with modifications to promote such resistance, for instance an oligonucleotide of the invention may have preferably been modified with a 2'- O-methyl group (e.g., 2'-0-methylcytidine, 2'-0-methylpseudouridine, 2'-0- methylguanosine, 2'-0-methyluridine, 2'-0-methyladenosine, 2'-0-methyl). In certain embodiments, the oligonucleotides contain a modification to increase resistance to ribonucleases and a phosphorothioate backbone.

In other embodiments, the oligonucleotides contain locked nucleic acids (oligonucleotides comprising at least one 2'-C,4'-C-oxy- methylene-linked bicyclic ribonucleotide monomer), 5-fluorouracil, 5-bromouracil, 5-chlorouracil, 5-iodouracil, hypoxanthine, xanthine, 4-acetylcytosine, 5-(carboxyhydroxylmethyl)uracil, 5-carboxymethylaminomethyl-2-thiouridine, 5-carboxymethylaminomethyluracil, dihydrouracil, beta-D-galactosylqueosine, inosine, N6-isopentenyladenine, 1- methylguanine, 1-methylinosine, 2,2-dimethylguanine, 2-methyladenine, 2-methylguanine, 3-methylcytosine, 5-methylcytosine, N6-adenine, 7-methylguanine, 5-methylaminomethyluracil, 5-methoxyaminomethyl-2-thiouracil, beta-D- mannosylqueosine, 5'-methoxycarboxymethyluraci 1, 5-methoxyuracil, 2-methylthio-N6- isopentenyladenine, uracil-5-oxyacetic acid (v), wybutoxosine, pseudouracil, queosine, 2- thiocytosine, 5-methyl-2-thiouracil, 2-thiouracil, 4-thiouracil, 5-methyluracil, uracil-5- oxyacetic acid methylester, uracil-5-oxyacetic acid (v), 5-methyl-2-thiouracil, 3-(3-amino- 3-N-2-carboxypropyl) uracil, (acp3)w, and 2,6-diaminopurine.

In some embodiments the oligonucleotide contains peptide nucleic acid (PNA), Morpholino nucleic acid, glycol nucleic acid (GNA), threose nucleic acid (TNA), hexitol nucleic acids (HNA).

In some embodiments, the oligonucleotides include modifications to the phosphate backbone such as methyl phosphonates, methyl phosphonothioates,
phosphoromorpholidates, phosphoropiperazidates and phosphoramidates. In some embodiments, the oligonucleotides contain a 2' loweralkyl moiety (e.g., C1-C4, linear or branched, saturated or unsaturated alkyl, such as methyl, ethyl, ethenyl, propyl, 1- propenyl, 2-propenyl, and isopropyl).

The oligonucleotides of the invention may be complementary to (and thus hybridize with) a region of the RNA transcript from the genes. In one embodiment, it is provided an oligonucleotide that is complementary to a target sequence of COL11A1 transcript mRNA. In another embodiment, it is provided an oligonucleotide that is complementary to a target sequence of MMP9 transcript mRNA. In another embodiment, it is provided an oligonucleotide that is complementary to a target sequence of COL5A2 transcript mRNA. In another embodiment, it is provided an oligonucleotide that is complementary to a target sequence of ITGB1 transcript mRNA. In another embodiment, it is provided an oligonucleotide that is complementary to a target sequence of TGFB1 transcript mRNA. In another embodiment, it is provided an oligonucleotide that is complementary to a target sequence of BMP1 transcript mRNA. In particular embodiments, the mRNA transcripts for each gene are those shown in table 2. By hybridizing with the target transcripts the oligonucleotide(s) of the present disclosure are able to reduce the expression of the encoded gene(s).

**Table 2. List of target mRNA transcripts**

| **Gene** | **Targeted Transcripts** |
|---|---|
| ***COL5A2*** | NM_000393.5 |
| ***COL11A1*** | NM_001854.4 |
| | NM_080629.3 |
| | NM_080630.4 |
| | NM_001190709.2 |
| ***MMP9*** | NM_004994.3 |
| ***ITGB1*** | NM_002211.4 NM_033668.2 |
| | NM_133376.3 |
| ***BMP1*** | NM_001199.4 |
| | NM_006129.5 |
| ***TGFB1*** | NM_000660.7 |

In one embodiment, the oligonucleotides will be complementary to 10, 11, 12, 13, 14, 15, 16, 17, 18, 19, 20, 21, 22, 23, 24, 25, 26, 27, 28, 29, or 30 nucleotides of the target sequence within the transcript, preferably complementary to 13-30 or 16-21 nucleotides of the target transcript.

In particular embodiments, the target sequence is comprised within the following regions:
- for COL5A2 the sequence delimited by the nucleotides in position 182 to 6410 in NM_000393.5 (SEQ ID NO: 1)
- for COL11A1 the sequence delimited by the nucleotides in position 486 to 5258 in NM_001854.4 (SEQ ID NO: 2)
- for MMP9 the sequence delimited by the nucleotides in position 379 to 2245 in NM_004994.3 (SEQ ID NO: 3)
- for ITGB1 the sequence delimited by the nucleotides in position 185 to 2927 in NM_002211.4 (SEQ ID NO: 4)
- for BMP1 the sequence delimited by the nucleotides in position 547 to 2378 in NM_001199.4 (SEQ ID NO: 5)
- for TGFB1 the sequence delimited by the nucleotides in position 1237 to 2575 in NM_000660.7 (SEQ ID NO: 6).

In particular embodiments, the target sequence for each transcript is comprised in a region defined by a sequence selected from those disclosed in tables 14-19 (i.e. the target sequence for COL5A2 is comprised within a sequence disclosed in table 14; the target sequence for COL11A1 is comprised within a sequence disclosed in table 15; the target sequence for MMP9 is comprised within a sequence disclosed in table 16; the target sequence for ITGB1 is comprised within a sequence disclosed in table 17; the target sequence for TGFB1 is comprised within a sequence disclosed in table 18; the target sequence for BMP1 is comprised within a sequence disclosed in table 19). In more particular embodiments, the target sequence for each transcript comprises or consists of a sequence selected from those defined in tables 14-19 (one table for each gene).

In one embodiment, the oligonucleotides of the first aspect are at least 10, 11, 12, 13, 14, 15, 16, 17, 18, 19, 20, 21, 22, 23, 24, 25, 26 or 27 nucleotides in length, preferably at least 23, 24, 25 or 26 nucleotides in length, for example 13-25 or 16-21 nucleotides in length. It may be that the region of the oligonucleotide capable of hybridizing is that length, or at least that length, but there are also additional nucleotides at the 5' and/or 3' ends of the oligonucleotide (overhangs), though in other instances the overall length of the oligonucleotide is that number of nucleotides. In general, oligonucleotide sequences which are 100% complementary to a portion of the target RNA may preferably be employed. In some instances, though, sequence variations that might be expected due to genetic mutation, strain polymorphism, or evolutionary divergence may be present. For example, oligonucleotide sequences with insertions, deletions, and single point mutations relative to the target sequence may also be effective for reducing the target gene expression. In particular embodiments the oligonucleotides are at least 80%, 81%, 82%, 83%, 84%, 85%, 86%, 87%, 88%, 89%, 90%, 91%, 92%, 93%, 94%, 95%, 96%, 97%, 98%, 99% or even 100% complementary to the target sequence within the mRNA transcript.

An oligonucleotide complementary to a certain target sequence is understood as an oligonucleotide having a sequence that will bind as a result of Watson-Crick base pairing to the target sequence. Said binding is also termed "hybridizing". Complementarity can be 100%, when all of the nucleotides bind to the target sequence, or less that 100%. Thus, the above embodiments can be alternatively expressed as oligonucleotides that comprise a sequence that has identity with respect to the reverse complimentary of a target sequence within a transcript mRNA. Identity can be of 100% or, alternatively, at least 80%, 81%, 82%, 83%, 84%, 85%, 86%, 87%, 88%, 89%, 90%, 91%, 92%, 93%, 94%, 95%, 96%, 97%, 98%, or 99% with respect to the reverse complimentary of the target sequence within the transcript mRNA.

Sequence identity, including determination of sequence complementarity for nucleic acid sequences, may be determined by sequence comparison and alignment algorithms known in the field. To determine the percent identity of two nucleic acid sequences (or of two amino acid sequences), the sequences are aligned for optimal comparison purposes (e.g., gaps can be introduced in the first sequence or second sequence for optimal alignment). The nucleotides (or amino acid residues) at corresponding nucleotide (or amino acid) positions are then compared. When a position in the first sequence is occupied by the same residue as the corresponding position in the second sequence, then the molecules are identical at that position. The percent identity between the two sequences is a function of the number of identical positions shared by the sequences (i.e., % homology=# of identical positions/total # of positions* 100), optionally penalizing the score for the number of gaps introduced and/or length of gaps introduced.

The comparison of sequences and determination of percent identity between two sequences can be accomplished using a mathematical algorithm. In one embodiment, the alignment generated over a certain portion of the sequence aligned having sufficient identity but not over portions having low degree of identity (i.e., a local alignment). A preferred, non- limiting example of a local alignment algorithm utilized for the comparison of sequences is the algorithm of Karlin and Altschul (1990) Proc. Natl. Acad. Sci. USA 87:2264-68, modified as in Karlin and Altschul (1993) Proc. Natl. Acad. Sci. USA 90:5873-77. Such an algorithm is incorporated into the BLAST programs (version 2.0) of Altschul, et al. (1990) J. Mol. Biol. 215:403-10. In another embodiment, the alignment is optimized by introducing appropriate gaps and percent identity is determined over the length of the aligned sequences (i.e., a gapped alignment). To obtain gapped alignments for comparison purposes, Gapped BLAST can be utilized as described in Altschul et al, (1997) Nucleic Acids Res. 25(17):3389-3402. In another embodiment, the alignment is optimized by introducing appropriate gaps and percent identity is determined over the entire length of the sequences aligned (i.e., a global alignment). A preferred, non-limiting example of a mathematical algorithm utilized for the global comparison of sequences is the algorithm of Myers and Miller, CABIOS (1989). Such an algorithm is incorporated into the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The oligonucleotides described herein can be single- stranded DNA or RNA, double- stranded DNA or RNA, DNA-RNA hybrids, or chimeric DNA-RNA structures. Examples of double-stranded RNA include, e.g., small interfering RNAs (siRNA), short hairpin RNA (shRNA) and other RNA interfering agents such as pre-miRNA. Single-stranded oligonucleotides include, e.g., antisense oligonucleotides, ribozymes, mature miRNA, and triplex- forming oligonucleotides. All of these can be prepared using standard methods.

Using known techniques and based on a knowledge of the sequence of the target gene, double-stranded RNA (dsRNA) molecules can be designed to reduce expression of the gene by sequence homology-based targeting of its RNA transcript. Such dsRNAs will typically be siRNAs, usually in a stem-loop ("hairpin") configuration, or microRNAs (miRNAs). The sequence of such dsRNAs will comprise a portion that is complementary to that of a portion of the target mRNA transcript. This portion will usually be 100% complementary to the target portion within the transcript but lower levels of complementarity (e.g. 80% or more, 85% or more, 90% or more, or 95% or more) may also be used.

Using known techniques and based on a knowledge of the sequence of the gene, single-stranded antisense oligonucleotides (AONs) can be designed to reduce expression of the target gene by sequence homology-based targeting of its RNA transcript. The sequence of such AONs will comprise a sequence that is complementary to that of a sequence of the target mRNA transcript. This portion will usually be 100% complementary to the target sequence within the transcript but lower levels of complementarity (e.g. 80% or more, 85% or more, 90% or more or 95% or more) may also be used. The siRNAs, miRNAs or AONs act by binding to target pre-mRNA or mRNA via Watson-Crick base pairing and inducing downregulation of gene expression by different mechanisms such as through mRNA cleavage, RNase H-mediated mRNA degradation or steric hindrance.

In one embodiment, the oligonucleotide is a small interfering RNA (siRNA). An siRNA acts by activating the RNAi-induced suppression complex. The siRNA molecules according to the present disclosure can be unmodified or modified and are capable of reducing gene expression. They are typically about 15 to 60 nucleotides in length, in particular from about 15 to 30 nucleotides in length, more particularly from about 18 to 25 nucleotides in length. In some embodiments, the siRNA contains modified nucleotides, for example, 2'0-Me purine or pyrimidine nucleotide, such as a 2'0-Me-guanosine, 2'O- Me-uridine, 2'0-Me-adenosine, and/or 2'0-Me-cytosine nucleotide. The modified nucleotides can be present in one strand (i.e., sense or antisense) or both strands of the siRNA. The modified siRNA may comprises from about 1% to about 100% (e.g., about 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 35%, 40%, 45%, 50%, 55%, 60%, 65%, 70%, 75%, 80%, 85%, 90%, 95%, or 100%) modified nucleotides in the double-stranded region of the siRNA duplex. In certain embodiments, one, two, three, four, five, six, seven, eight, nine, ten, or more of the nucleotides in the double-stranded region of the siRNA comprise modified nucleotides. The siRNA sequences may have overhangs or blunt ends. In a particular embodiment, the siRNA sequences have overhangs.

Suitable siRNA sequences can be identified using any means known in the art. In particular embodiments, the oligonucleotides are siRNAs selected from those shown in table 20.

The oligonucleotides of the disclosure can be synthesized using any of a variety of techniques known in the art. In one embodiment, the oligonucleotides are chemically synthesized. The synthesis of oligonucleotides makes use of common nucleic acid protecting and coupling groups, such as dimethoxytrityl at the 5 '-end and phosphoramidites at the 3 '-end. Double stranded oligonucleotides, such as siRNA molecules, can be assembled from two distinct single stranded oligonucleotides, wherein one oligonucleotide comprises the sense strand and the other comprises the antisense strand of the siRNA. For example, each strand can be synthesized separately and joined together by hybridization or ligation following synthesis and/or deprotection. In certain other instances, siRNA molecules can be synthesized as a single continuous oligonucleotide fragment, where the self-complementary sense and antisense regions hybridize to form an siRNA duplex having hairpin secondary structure.

In other embodiments the oligonucleotides are miRNAs. In particular embodiments, said miRNAs are selected from those the list consisting of hsa-miR-143-3p (MIMAT0000435), hsa-miR-223-3p (MIMAT0000280), hsa-miR-574-5p (MIMAT0004795), hsa-miR-142-3p (MIMAT0000434), hsa-miR-452-5p (MIMAT0001635), hsa-miR-145-5p (MIMAT0000437), hsa-miR-145-3p (MIMAT0004601), hsa-miR-224-5p (MIMAT0000281), hsa-miR-338-5p (MIMAT0004701), hsa-miR-542-3p (MIMAT0003389), hsa-miR-365a-3p (MIMAT0000710), hsa-miR-365b-3p (MIMAT0022834), hsa-miR-335-5p (MIMAT0000765), hsa-miR-30a-5p (MIMAT0000087), hsa-miR-199b-3p (MIMAT0000232), hsa-miR-199a-3p (MIMAT0000232), hsa-miR-223-5p (MIMAT0004570), hsa-miR-152-3p (MIMAT0000438), and hsa-miR-497-5p (MIMAT0002820). References in brackets refer to the accession number of the miRNAs according to miRBase database (Release 22.1).

In some embodiments, the oligonucleotide is an AON. An AON consists of a short, single-stranded DNA or RNA molecule with or without potential modifications to both the backbone and sugar rings that render the molecule resistant to nuclease degradation, more stable in plasma and less immunogenic. DNA AONs act in the nucleus where they bind to their target transcript, forming a DNA/RNA complex which is recognized by the enzyme RNase H and results in mRNA degradation. AONs may, also, block protein translation by binding the target-transcript and preventing ribosomal attachment, without recruitment of the RNase H. Another mechanism of AONs function involves binding to regulatory sequences within and surrounding the exon/intron boundaries and thus, altering pre-mRNA splicing (i.e., removal of introns to produce mature mRNA). In certain embodiments, the AONs can be gapmers or altimers. A gapmer is a chimeric AON that contains a central block of DNA molecules and is flanked by blocks of 2'-0 modified ribooligonucleotides or other artificially modified ribooligonucleotide monomers that protect the internal block from nuclease degradation. In a particular embodiment, the AON comprises modifications to help enhance the properties of the oligonucleotide

In one embodiment, the oligonucleotide is a guide RNA comprising a guide RNA sequence and a tracr RNA. The guide RNA sequence is capable of hybridizing to a target sequence. The tracr RNA is coupled to the guide RNA sequence. The guide RNA hybridises to the target site and targets a CRISPR-Cas enzyme to said site. In some embodiments, the guide sequence is between 10-30, or between 15-25, or between 15-20 nucleotides in length. Preferably the CRISPR- Cas enzyme is a Type II CRISPR enzyme, for example Cas-9. The enzyme complexes with the guide RNA. In one embodiment, the complex is targeted to the DNA sequence of the gene of interest, i.e. COL5A2, COL11A1, MMP9, ITGB1, TGFB1, or BMP1, and will bind by hybridization. In one embodiment, the enzyme is active and acts as an endonuclease to cleave the DNA either via activation of the non- homologous end-joining or homologous DNA repair pathway, resulting in a blunt end cut or a nick. A repair template sequence can be supplied and be introduced into the gene by homologous recombination, thereby replacing the sequence that it targeted. In another embodiment, the enzyme is targeted to the DNA of the gene of interest but the enzyme comprises one or more mutations that reduce or eliminate its endonuclease activity such that it does not edit the gene but does prevent or reduce its transcription. In another embodiment, the enzyme can be engineered such that it is fused to a transcriptional repressor to reduce or disable its endonuclease function. The enzyme will be able to bind the guide RNA and be targeted to the DNA sequence, but no cleavage of the DNA takes place. The gene of interest may be suppressed, for example, by the shutting down of the promoter or blockage of RNA polymerase. In another embodiment, the transcription repressor may be bound to the tracr sequence. Functional domains can be attached to the tracr sequence by incorporating protein-binding RNA aptamer sequences, as described in Konermann et al (Nature volume 517, pages 583-588 (2015)). The transcription repressor-tracr sequence complex may be used to target other moieties to a precise gene location as desired.

The oligonucleotides (e.g., siRNAs, miRNAs, or AONs) may be provided on their own or together with a gene vector or carrier, or together with other molecules which contribute to the desired therapeutic effect. The inclusion of the oligonucleotide disclosed herein within a viral or non-viral vector, or its combination with a nanoparticle, vesicle, or any other carrier, may be convenient, for example, in order to target the desired cells or tissues, increase transfection efficiency and/or improve degradation resistance. Non-limited carriers which are appropriate for delivering the oligonucleotides of the present disclosure are polycationic polymers. In other embodiments, the oligonucleotides may be conjugated with a peptide or receptor. To assist with delivery of the oligonucleotide, the peptide may for example be a cell penetrating peptide.

### Combinations

As outlined above, the present disclosure refers to a combination of oligonucleotides, wherein said combination reduces the expression of a gene selected from the group consisting of COL5A2, COL11A1, MMP9, ITGB1, TGFB1 and BMP1. In one embodiment, the combination comprises at least two oligonucleotides, each reducing the expression of a different gene selected from the group consisting of COL5A2, COL11A1, MMP9, ITGB1, TGFB1 and BMP1. In another embodiment, the combination comprises at least three oligonucleotides, each reducing the expression of a different gene selected from the group consisting of COL5A2, COL11A1, MMP9, ITGB1, TGFB1 and BMP1. In another embodiment, the combination comprises at least four oligonucleotides, each reducing the expression of a different gene selected from the group consisting of COL5A2, COL11A1, MMP9, ITGB1, TGFB1 and BMP1. In another embodiment, the combination comprises at least five oligonucleotides, each reducing the expression of a different gene selected from the group consisting of COL5A2, COL11A1, MMP9, ITGB1, TGFB1 and BMP1. In one embodiment, the combination comprises at least one oligonucleotide that reduces the expression of COL11A1. In another embodiment, the combination comprises at least one oligonucleotide that reduces the expression of MMP9. In another embodiment, the combination comprises at least one oligonucleotide that reduces the expression of COL5A2. In another embodiment, the combination comprises at least one oligonucleotide that reduces the expression of ITGB1. In another embodiment, the combination comprises at least one oligonucleotide that reduces the expression of TGFB1. In another embodiment, the combination comprises at least two oligonucleotides, one reducing the expression of COL11A1 and the other one reducing the expression of MMP9. In a particular embodiment, the combination comprises at least one further oligonucleotide, said further oligonucleotide reducing the expression of ITGB1. In a more particular embodiment, the combination comprises at least six oligonucleotides, each reducing the expression of a different gene selected from the group consisting of COL5A2, COL11A1, MMP9, ITGB1, TGFB1 and BMP1.

All embodiments described in the section above for the oligonucleotides also apply to the combinations disclosed herein. In a particular embodiment, the combination comprises six oligonucleotides, each of which hybridize (comprise a sequence that is complementary to) with a different transcript of those shown in table 2. In a more particular embodiment, the target region within each transcript is comprised in a region defined by a sequence selected from those disclosed in tables 14-19. In a more particular embodiment, the oligonucleotides are 15-30, or 18-25 nucleotides long. In a more particular embodiment, the oligonucleotides are siRNAs. In an even more particular embodiment, the siRNAs are those shown in table 20.

### Compositions

A second aspect of this disclosure refers to a composition comprising a combination as defined above. All embodiments described above for the combinations and oligonucleotides also apply to the composition of the second aspect.

In some embodiments, the composition is a pharmaceutical composition comprising a therapeutically effective amount of the oligonucleotide composition together with pharmaceutically acceptable excipients and/or carriers.

The expression "pharmaceutically acceptable excipients or carriers" refers to pharmaceutically acceptable materials, compositions or vehicles. Each component must be pharmaceutically acceptable in the sense of being compatible with the other ingredients of the pharmaceutical composition. It must also be suitable for use in contact with the tissue or organ of humans and animals without excessive toxicity, irritation, allergic response, immunogenicity or other problems or complications commensurate with a reasonable benefit/risk ratio.

The election of the pharmaceutical formulation will depend upon the nature of the active compound and its route of administration. Any route of administration may be used. In some embodiments, the route of administration is parenteral, and the composition is then appropriate for parenteral administration. In a particular embodiment, the route of administration is by injection. In a more particular embodiment, the route of administration is systemic, for example, by intramuscular, intravenous, intraarterial, intraperitoneal, subcutaneous, or transdermal injection. In a particular embodiment, the route of administration is local, for example, intratumoral injection. Topical administration is also contemplated, such that the pharmaceutical composition may be a topical composition.

The pharmaceutical compositions may be in any form, including, among others, tablets, pellets, capsules, aqueous or oily solutions, suspensions, emulsions, aerosols, or dry powdered forms suitable for reconstitution with water or other suitable liquid medium before use, for immediate or retarded release.

The appropriate excipients and/or carriers, and their amounts, can readily be determined by those skilled in the art according to the type of formulation being prepared. Examples of suitable pharmaceutically acceptable excipients are solvents, dispersion media, diluents, or other liquid vehicles, dispersion or suspension aids, surface active agents, isotonic agents, thickening or emulsifying agents, preservatives, solid binders, lubricants and the like. Except insofar as any conventional excipient medium is incompatible with a substance or its derivatives, such as by producing any undesirable biological effect or otherwise interacting in a deleterious manner with any other component(s) of the pharmaceutical composition, its use is contemplated to be within the scope of this disclosure.

The term "carrier" is to be understood as a pharmaceutically acceptable vehicle. The carrier can be organic, inorganic, or both. Suitable carriers well known to those of skill in the art and include, without limitation, large, slowly metabolized macromolecules such as proteins, polysaccharides, polylactic acids, polyglycolic acids, polymeric amino acids, amino acid copolymers, lipid aggregates (such as oil droplets or liposomes) and inactive virus particles. Carriers may also include, saline, buffer, dextrose, water, glycerol, ethanol, and the combinations thereof. In particular embodiments, the carries may be a polycationic polymer, a vesicle, a liposome, or a nanoparticle.

In one particular embodiment, the pharmaceutical composition is a sterile injectable solution. This may be prepared, for instance, by incorporating the oligonucleotides in the required amount in an appropriate solvent with one or a combination of ingredients enumerated above, as required, followed by filtered sterilization. In the case of sterile powders for the preparation of sterile injectable solutions, preferred methods of preparation include vacuum drying and freeze-drying which yields a powder of the active ingredient plus any additional desired ingredient from a previously sterile-filtered solution thereof.

Usually, the pharmaceutical composition comprises a therapeutically effective amount of the oligonucleotides. The expression "therapeutically effective amount" as used herein, refers to the amount of a compound that, when administered, is sufficient to prevent development of, or alleviate to some extent, one or more of the symptoms of the disease which is addressed. The particular dose of compound administered according to this disclosure will of course be determined by the particular circumstances surrounding the case, including the compound administered, the route of administration, the particular condition being treated, and the similar considerations. The therapeutically effective amount may be 1mg to 800mg of each oligonucleotide. The amount of oligonucleotide administered (or dose) may be from about 1 microgram/kilogram body weight to about 10 milligrams/kilogram body weight per day, in particular the dose may be 100mg. In addition, the nanobodies, nanobody conjugates, vectors or viral particles of the present disclosure may also be used with other therapeutic agents.

In a particular embodiment, the compositions of the second aspect further comprise heparin, a heparin surrogate, or antibodies that bind to the heparin binding domain within the COL5A2, COL11A1, MMP9, ITGB1, TGFB1 and BMP1 proteins.

In one embodiment the composition comprises heparin. In the sense of the present disclosure, the term "heparin" encompasses heparin surrogates, also called heparin mimetics. "Heparin surrogate" or "heparin mimetic" is understood by the skilled person as an anionic species that binds to a heparin-binding site within a protein. They usually are synthetic and semi-synthetic compounds that are highly sulfated, structurally distinct analogues of glycosaminoglycans. Non-limiting examples of heparin mimetics are polyvinyl sulfonate (PVS), Fondaparinux, Indraparinux, Idrabiotaparinux, etc. Other heparin derivatives include heparin-like glycosaminoglycans (HLGAGs), sulfated non-anticoagulant heparin (S-NACH), low-molecular-weight heparintaurocholate-tetramer deoxycholate (LHTD4), LHTD4/DCK (a complex of LHTD4 and deoxycholylethylamineDCK), high-molecular-weight Escherichia coli K5-derived heparin-like polysaccharide (K5-NSOS), LHsura (a complex of heparin and suramin fragment), and LHbisD4 (a conjugation of low molecular weight heparin and four bis-deoxycholates). In a particular embodiment, the heparin is low molecular weight heparin (LMWH).

As outlined above, the inventor has found that several heparin-binding domains are present withing the COL5A2, COL11A1, MMP9, ITGB1, TGFB1 and BMP1 proteins. Some aspects of the invention therefore refer to one or more protein inhibitors, which, according to the present disclosure, are compounds that specifically bind to the proteins of interest (i.e., COL5A2, COL11A1, MMP9, ITGB1, TGFB1 and/or BMP1) at the site of the heparin binding domains. The heparin-binding domain for each of the proteins of interest is shown in tables 3 through 13. In particular embodiments, it is provided a protein inhibitor that binds to a sequence selected from those defined in tables 3 to 13. Said protein inhibitors are able to block the heparin binding domains, preventing thus, the interactions with other proteins and receptors such as integrins resulting in stabilization and decreased degradation of the ECM, and thus, exerting anti-metastatic activity.

**Table 3: Putative heparin binding sites within the COL5A 1 protein.**

| **Docking model #** | **Heparin binding site residues** | **Position in amino acid sequence** | **Protein region/domain** |
|---|---|---|---|
| 1 | KGRR (SEQ ID NO: 7) | 605, 607-609 | Chain, triple helical region |
| 2 | KPGRR (SEQ ID NO: 8) | 605-609 | Chain, triple helical region |
| 3 | GRSKRKK (SEQ ID NO: 9) | 1689, 1691, 1694, 1697, 1709, 1711, 1826 | C-terminal propeptide, fibrillar collagen NC1 domain |
| 4 | RTWKKRK (SEQ ID NO: 10) | 1691, 1693, 1695, 1697, 1708, 1709, 1711 | C-terminal propeptide, fibrillar collagen NC1 domain |
| 5 | GHRGGWP (SEQ ID NO: 11) | 643-645, 649, 994, 998, 999, 1001 | Chain, triple helical region |
| 6 | KGRRR (SEQ ID NO: 12) | 605, 607-609, 611 | Chain, triple helical region |

**Table 4: Putative heparin binding sites within the COL5A2 protein.**

| **Docking model #** | **Heparin binding site residues** | **Position in amino acid sequence** | **Protein region/domain** |
|---|---|---|---|
| 1 | NNIRRRQNR (SEQ ID NO: 13) | 1429, 1438, 1439, 1440, 1442, 1465, 1467, 1468, 1471 | C-terminal propeptide, fibrillar collagen NC1 domain |
| 2 | RGRKG (SEQ ID NO: 14) | 109-113 | Chain region |
| 3 | RGRK (SEQ ID NO: 15) | 109-112 | Chain region |
| 4 | SPDNKVQKKRW (SEQ ID NO: 16) | 1352, 1355, 1356, 1357, 1358, 1360, 1415, 1420, 1421, 1453, 1480, 1482 | C-terminal propeptide, fibrillar collagen NC1 domain |
| 5 | WSKSPDNKVKRVDVGTQ (SEQ ID NO: 17) | 1350, 1352-1358, 1360, 1421, 1453, 1480-1482, 1484, 1485, 1487 | C-terminal propeptide, fibrillar collagen NC1 domain |

**Table 5: Putative heparin binding sites within the COL11A1 protein.**

| **Docking model #** | **Heparin binding site residues** | **Position in amino acid sequence** | **Protein region/domain** |
|---|---|---|---|
| 1 | KKRR (SEQ ID NO: 18) | 575, 578, 579, 581 | Chain triple-helical region, collagen-like 2 domain |
| 2 | RWKTKRW (SEQ ID NO: 19) | 7-13 | Signal peptide region |
| 3 | SKKTRR (SEQ ID NO: 20) | 1551-1556 | Chain nonhelical region (C-terminal) |
| 4 | LLFRYQRL (SEQ ID NO: 21) | 493, 495, 497-499, 517, 524, 527 | N-terminal propeptide & Telopeptide chain region |
| 5 | KKRGR (SEQ ID NO: 22) | 575, 578-581 | Chain triple-helical region, collagen-like 2 domain |

**Table 6: Putative heparin binding sites within the ITGA3 protein.**

| **Docking model #** | **Heparin binding site residues** | **Position in amino acid sequence** | **Protein region/domain** |
|---|---|---|---|
| 1 | RRRR (SEQ ID NO: 23) | 872-875 | Interchain between ITGA3 heavy and light chain - Disulfide bond |
| 2 | RNRRRAY (SEQ ID NO: 24) | 510, 511, 579, 585, 589, 593, 594 | ITGA3 heavy chain - Extracellular region |
| 3 | RLTARRRRRGLRAY (SEQ ID NO: 25) | 38, 40, 97, 98, 461, 510, 579, 583, 585, 587-589, 593, 594 | ITGA3 heavy chain - Extracellular region |

**Table 7: Putative heparin binding sites within the ITGA4 protein.**

| **Docking model #** | **Heparin binding site residues** | **Position in amino acid sequence** | **Protein region/domain** |
|---|---|---|---|
| 1 | SEEEKQKRKMK (SEQ ID NO: 26) | 791, 794, 796, 798, 867, 871, 874, 878, 882, 928, 973 | ITGA4 chain - Extracellular region |
| 2 | SNQKTKGRFKHHQRK (SEQ ID NO: 27) | 791, 792, 865, 867, 872, 874, 875, 878, 879, 882, 968, 969-971, 973 | ITGA4 chain - Extracellular region |
| 3 | RRR (SEQ ID NO: 28) | 7, 12, 13 | Signal peptide |
| 4 | GRRT (SEQ ID NO: 29) | 10, 12, 13, 19 | Signal peptide |
| 5 | PGRRR (SEQ ID NO: 30) | 9, 10, 12, 13, 17 | Signal peptide |
| 6 | SQKQKGRKHRK (SEQ ID NO: 31) | 791, 865, 867, 871, 874, 875, 878, 882, 969, 971, 973 | ITGA4 chain - Extracellular region |

**Table 8: Putative heparin binding sites within the ITGA6 protein.**

| **Docking model #** | **Heparin binding site residues** | **Position in amino acid sequence** | **Protein region/domain** |
|---|---|---|---|
| 1 | RKKKSKT (SEQ ID NO: 32) | 19, 565, 569, 586, 624, 652, 653 | Signal peptide, Disulfide bond, ITGA6 heavy chain - Extracellular region |
| 2 | DRSRRRVNSLP (SEQ ID NO: 33) | 31, 91, 631, 633-640 | ITGA6 heavy chain - Extracellular region |
| 3 | REKRKKKT (SEQ ID NO: 34) | 19, 562, 565, 568, 569, 586, 652, 653 | Signal peptide, Disulfide bond, ITGA6 heavy chain - Extracellular region |
| 4 | RGKKKSKT (SEQ ID NO: 35) | 19, 21, 459, 565, 586, 624, 652, 653 | Signal peptide, Disulfide bond, ITGA6 heavy chain - Extracellular region |

**Table 9: Putative heparin binding sites within the ITGB1 protein.**

| **Docking model #** | **Heparin binding site residues** | **Position in amino acid sequence** | **Protein region/domain** |
|---|---|---|---|
| 1 | RKLKRDRNKR (SEQ ID NO: 36) | 98, 105-107, 174, 179, 181, 227, 228, 268 | ITGB1 chain - Extracellular region |
| 2 | RKKRSDRNKR (SEQ ID NO: 37) | 98, 105, 107, 174, 178, 179, 181, 227, 228, 268 | ITGB1 chain - Extracellular region |
| 3 | NSAAKKKRTNEYS (SEQ ID NO: 38) | 25, 61, 62, 69, 72, 73, 540, 541, 544-546, 548, 549 | ITGB1 chain - Extracellular region |
| 4 | RSKGTKKRSDRNKR (SEQ ID NO: 39) | 98-102, 105, 107, 174, 178, 179, 181, 227, 228, 268 | ITGB1 chain - Extracellular region |
| 5 | NKGTSGRVQCVCKK (SEQ ID NO: 40) | 31, 33, 57, 60, 61,497, 498, 532, 535-538, 540, 551 | ITGB1 chain - Extracellular region |
| 6 | NQGPSAAKKCKRTNEYGN (SEQ ID NO: 41) | 25, 55, 57, 59, 61, 62, 69, 72, 73, 538, 540, 541, 544, 545, 546, 548, 550, 557 | ITGB1 chain - Extracellular region |
| 7 | PKRNSNTSKR (SEQ ID NO: 42) | 200, 202, 204, 205, 209, 212, 214, 215, 220, 240 | ITGB1 chain - Extracellular region, CX3CL1-binding |

**Table 10: Putative heparin binding sites within the MMP2 protein.**

| **Docking model #** | **Heparin binding site residues** | **Position in amino acid sequence** | **Protein region/domain** |
|---|---|---|---|
| 1 | WKNKKYRNKKMDP (SEQ ID NO: 43) | 574, 576-579, 581, 590, 592, 595, 597-600 | Region required for inhibitor TIMP2 binding, Hemopexin 3 repeat |
| 2 | NKKYRNKK (SEQ ID NO: 44) | 577-579, 581, 590, 592, 595, 604 | Region required for inhibitor TIMP2 binding, Hemopexin 3 repeat |
| 3 | KNKYRNEVKK (SEQ ID NO: 45) | 576, 577, 579, 581, 590, 592-595, 604 | Region required for inhibitor TIMP2 binding, Hemopexin 3 repeat |

**Table 11: Putative heparin binding sites within the MMP9 protein.**

| **Docking model #** | **Heparin binding site residues** | **Position in amino acid sequence** | **Protein region/domain** |
|---|---|---|---|
| 1 | YRRVARRKKMVR (SEQ ID NO: 46) | 50, 51, 56-58, 61, 599, 603, 623, 641, 642, 645 | Activation propeptide, MMP9 chain |
| 2 | RVAKQRDKGLGAWR (SEQ ID NO: 47) | 56-58, 65, 108, 600, 602, 603, 605-608, 633, 645 | Activation propeptide, MMP9 chain |
| 3 | RSRKRK (SEQ ID NO: 48) | 61, 619, 621, 623, 634, 638 | MMP9 chain |
| 4 | RKRDRR (SEQ ID NO: 49) | 61, 623, 634, 643, 645, 652 | MMP9 chain |
| 5 | RRRKRK (SEQ ID NO: 50) | 574, 618, 621, 623, 634, 638 | MMP9 chain |
| 6 | YRVAYRKGGRW (SEQ ID NO: 51) | 50, 56-58, 179, 599, 603, 605, 607, 631, 633 | Activation propeptide, MMP9 chain |
| 7 | YRVARRKGADRWP (SEQ ID NO: 52) | 50, 56, 57, 58, 61, 599, 603, 607-609, 631, 633, 644 | Activation propeptide, MMP9 chain |

**Table 12: Putative heparin binding sites within the TGFB1 protein.**

| **Docking model #** | **Heparin binding site residues** | **Position in amino acid sequence** | **Protein region/domain** |
|---|---|---|---|
| 1 | RRRSTKNW (SEQ ID NO: 53) | 275, 277, 278, 287, 289, 291, 292, 330 | Latency-associated peptide, Cleavage site by Furin, MMP9 Chain |
| 2 | RRRTKNW (SEQ ID NO: 54) | 275, 277, 278, 289, 291, 292, 330 | Latency-associated peptide, Cleavage site by Furin, MMP9 Chain |
| 3 | RRRTKNPYIWQ (SEQ ID NO: 55) | 275, 277, 278, 289, 291, 292, 327-330, 345 | Latency-associated peptide, Cleavage site by Furin, MMP9 Chain |
| 4 | TKKGTTRAHR (SEQ ID NO: 56) | 116, 161, 163, 238, 240, 241, 243, 248, 251, 255 | Latency-associated peptide |
| 5 | THKKDGTTGRLAHR (SEQ ID NO: 57) | 116, 117, 125, 161, 235, 238, 240-243, 247, 248, 251, 255 | Latency-associated peptide |
| 6 | TFKQSGFTTRR (SEQ ID NO: 58) | 116, 124-127, 238-241, 243, 255 | Latency-associated peptide |
| 7 | ETHFKSIYGFTGRR (SEQ ID NO: 59) | 115-117, 124, 125, 127, 131, 132, 238, 239, 241, 242, 243, 255 | Latency-associated peptide |

**Table 13: Putative heparin binding sites within the BMP1 protein.**

| **Docking model #** | **Heparin binding site residues** | **Position in amino acid sequence** | **Protein region/domain** |
|---|---|---|---|
| 1 | RGRPRK (SEQ ID NO: 60) | 113, 114, 117, 228, 232, 251 | Propeptide, |
| | | | Peptidase M12A domain, BMP1 chain |
| 2 | GRSRSRRR (SEQ ID NO: 61) | 114-120, 232 | Propeptide |
| 3 | RGRSRR (SEQ ID NO: 62) | 113-117, 232 | Propeptide |
| 4 | SRSRRRDDRHKEYK (SEQ ID NO: 63) | 116-120, 227, 229, 231-233, 251, 262, 264, 289 | Propeptide, |
| | | | Peptidase M12A domain, BMP1 chain |
| 5 | RSRRRKQSLG (SEQ ID NO: 64) | 117-120, 232, 251, 255, 259-261 | Propeptide, |
| | | | Peptidase M12A domain, BMP1 chain |
| 6 | QRSRPDRRRFKE (SEQ ID NO: 65) | 70, 117, 118, 120, 228-230, 232, 238, 249, 251, 253 | Propeptide, |
| | | | Peptidase M12A domain, BMP1 chain |
| 7 | SRSRRRPRKYKY (SEQ ID NO: 66) | 116-120, 227, 228, 232, 251, 264, 289, 290 | Propeptide, |
| | | | Peptidase M12A domain, BMP1 chain |

In some embodiments, the protein inhibitors are selected from polypeptides, aptamers, antibodies, antibody fragments, and antibody variants (such as nanobodies, scFV, bispecific antibodies, BiTEs). In a particular embodiment the protein inhibitor is a polypeptide of a size spectrum of approximately 8-45 amino acids. Peptide-based drugs are characterized by high degree of specificity to their in vivo targets, resulting in exceptionally high potency of action and relatively few off-target side effects. An approach of peptide-based therapy is to disrupt protein-protein interactions (PPIs) with common protein binding sites. Binding of the protein inhibitors to PPI interfaces lead to inhibition of downstream signalling.

In certain embodiments the protein inhibitor is a polypeptide chemically modified to increase its stability and/or decrease immunogenicity. Non-limitative methods to enhance proteolytic stability and to decrease immunogenicity of peptides include peptide cyclization, conjugation of peptides to natural or synthetic polymers and substitution of L-amino acids with D-amino acids.

In other embodiments the protein inhibitors are antibodies, fragments, or variants thereof, that bind to the heparin-binding domain within the proteins COL5A2, COL11A1, MMP9, ITGB1, TGFB1 and/or BMP1, as defined above.

In particular embodiments, it is provided a combination of protein inhibitors, wherein the combination contains at least as many protein inhibitors that specifically bind to the proteins of interest (i.e., COL5A2, COL11A1, MMP9, ITGB1, TGFB1 and/or BMP1) as required for binding to each and every one of these proteins.

### Kits

Herein disclosed is also a kit of parts that comprises at least one oligonucleotide as defined above. In particular embodiments the kit comprises more than one oligonucleotide as defined above. In particular embodiments, the kit comprises at least one oligonucleotide that reduces the expression of COL11A1. In other particular embodiments, the kit comprises at least one oligonucleotide that reduces the expression of MMP9. In other particular embodiments, the kit comprises at least one oligonucleotide that reduces the expression of COL5A2. In other particular embodiments, the kit comprises at least one oligonucleotide that reduces the expression of ITGB1. In other particular embodiments, the kit comprises at least one oligonucleotide that reduces the expression of TGFB1. In other particular embodiments, the kit comprises at least one oligonucleotide that reduces the expression of BMP1. In a more particular embodiment, the kit comprises at least two oligonucleotides, one reducing the expression of COL11A1 and the other one reducing the expression of MMP9. In a more particular embodiment, the kit comprises at least six oligonucleotides, each reducing the expression of a different gene selected from the group consisting of COL5A2, COL11A1, MMP9, ITGB1, TGFB1 and BMP1. All embodiments described above for the combinations and oligonucleotides also apply to the kits.

In some embodiments, the kit further comprises heparin, a heparin surrogate, heparin derivative or one or more protein inhibitors as defined in the fifth aspect of the disclosure. In other embodiments, the kit comprises instructions for its use in the treatment of cancer. All embodiments described above for the heparin, heparin surrogates and one or more protein inhibitors also apply to the kits.

### Medical uses

A third aspect of the present disclosure is directed to an oligonucleotide, combination of oligonucleotides, or composition, as defined above, for use as a medicament, in particular, for treating cancer. All embodiments defined above for the oligonucleotides, combinations and compositions also apply to their uses.

As used herein the terms "cancer" or "tumor" are used interchangeably and refer as generally understood in the art to a malignant abnormal cell growth with the potential to invade or spread to other parts of the body. As used herein, the term "treating" includes any of following: the prevention of the disease or disorder or of one or more symptoms associated with the disease or disorder; a reduction or prevention of the development or progression of the disease or disorder or symptoms; and the reduction or elimination of an existing disease or disorder or symptoms.

In particular, the treatment of cancer according to the present disclosure may be achieved by reducing or supressing cancer cell migration and invasiveness. Thus, in one embodiment the oligonucleotides, combination of oligonucleotides or compositions as defined above are for use in reducing or supressing cancer metastasis. In other particular embodiments, they are for use in treating metastatic cancer. In other particular embodiments, they are for use in patients having documented resistance to chemotherapy or immunotherapy. In other embodiments, they are for use in patients presenting with relapse following cancer treatment.

The cancer which can be treated according to the tenth aspect is not particularly limited. Non-limiting types of tumors or cancers that can be treated in the sense of the present description are carcinomas, sarcomas, and hematologic tumors. For example, the cancer to be treated may be a carcinoma, such as adenocarcinoma, sarcoma, osteosarcoma, chondrosarcoma, leiomyosarcomas, rhabdomyosarcomas, mesotheliomas, fibrosarcomas, angiosarcomas, liposarcomas, gliomas, myxosarcomas, mesenchymous, a hematologic cancer, such as myeloma, leukemia, or lymphoma, and mixed type tumors, such as adenosquamous carcinomas, mixed mesodermal tumors, carcinosarcomas, or teratocarcinomas. Carcinomas are the predominant cancers and are cancers of epithelial cells or cells covering the external or internal surfaces of organs, glands, or other body structures (e.g., skin, uterus, lung, breast, prostate, stomach, bowel), and which tend to metastasize. Carcinomas may be adenocarcinomas, for example, of the breast, lung, colon, prostate or bladder, and squamous cell carcinomas. Sarcomas are derived from connective or supportive tissue (e.g., bone, cartilage, tendons, ligaments, fat, and muscle). Sarcomas may be osteosarcomas or osteogenic sarcomas (bone), chondrosarcomas (cartilage), leiomyosarcomas (smooth muscle), rhabdomyosarcomas (skeletal muscle), mesothelial sarcomas or mesotheliomas (membranous lining of body cavities), fibrosarcomas (fibrous tissue), angiosarcomas or hemangioendotheliomas (blood vessels), liposarcomas (adipose tissue), gliomas or astrocytomas (neurogenic connective tissue found in the brain), myxosarcomas (primitive embryonic connective tissue), or mesenchymous or mixed mesodermal tumors (mixed connective tissue types). Hematologic tumors are derived from bone marrow and lymphatic tissue. Hematologic tumors may be myelomas, which originate in the plasma cells of bone marrow; leukemias which may be "liquid tumors" and are tumors of the bone marrow and may be myelogenous or granulocytic leukemia (myeloid and granulocytic white blood cells), lymphatic, lymphocytic, or lymphoblastic leukemias (lymphoid and lymphocytic blood cells) or polycythemia vera or erythremia (various blood cell products, but with red cells predominating); or lymphomas, which may be solid tumors and which develop in the glands or nodes of the lymphatic system, and which may be Hodgkin or Non-Hodgkin lymphomas. In addition, mixed type tumors, such as adenosquamous carcinomas, mixed mesodermal tumors, carcinosarcomas, or teratocarcinomas also exist. All these types of tumors, as well as others that would be apparent to the skilled person, are contemplated in the sense of the present disclosure.

Tumors that may be treated in the sense of the present disclosure may also be named based on the organ in which they originate i.e., the "primary site," for example, tumor, or cancer, of the breast, brain, lung, liver, skin, prostate, testicle, bladder, colon and rectum, cervix, uterus, blood, lymph, etc. This naming often persists even if the tumor metastasizes to another part of the body that is different from the primary site. In this sense, the cancer contemplated in the tenth aspect may be, but is not limited to, a cancer of the gastrointestinal tract, respiratory system, nervous system, hematopoietic system, epitelium, vascular system, reproductive system (such as cervical, ovarian, uterine, vaginal, and vulvar), urinary tract, endocrine system, skin, heart, brain, eyes, testes, muscles, bones or breasts.

In particular embodiments the cancer to be treated is a solid tumor. In particular embodiments the cancer is non-small cell lung cancer, breast cancer, colon cancer, rectal cancer, small intestine cancer, prostate cancer, small cell lung cancer, mesothelioma, kidney cancer, pancreatic cancer, stomach cancer, esophageal cancer, laryngeal cancer, oropharyngeal cancer, liver cancer, bile duct cancer, gallbladder cancer, bladder cancer, thyroid cancer, endometrial cancer, ovarian cancer, vaginal cancer, urethral cancer, testicular cancer, bone cancer, brain cancer, skin cancer, melanoma, sarcoma, angiosarcoma, liposarcoma etc. In particular embodiments the cancer is osteosarcoma or adenocarcinoma.

When used in combined therapy, the oligonucleotides, combination of oligonucleotides or compositions as defined above may be combined with heparin, a heparin surrogate, including antibodies binding to the heparin binding domain within the proteins of interest as described above. The combined therapy may alternatively or additionally be with an antitumoral agent, with radiotherapy or with surgery. The oligonucleotides, combination of oligonucleotides or compositions as defined above may be administered concurrently, sequentially, or separately, in any order within a therapeutically effective interval, with the heparin, heparin surrogate, one or more protein inhibitors, antitumoral agent, radiotherapy and/or with surgery. In a particular embodiment the combined therapy comprises surgery. In another particular embodiment the combined therapy comprises radiation. In a particular embodiment the combined therapy comprises an antitumoral agent. The antitumoral agent may be a cytotoxic agent, such as, for example, alkylating agents, antimetabolites, including folate antagonists, purine and pyrimidine analogues, antibiotics and other natural products, including anthracyclines and vinca alkaloids, and antibodies, which improve specificity. The antitumoral agent may also be a hormonal agent or a signal transduction inhibitor. In a particular embodiment the combination therapy comprises chemotherapy. The combined therapy may also comprise a different immunotherapy. For example, in a particular embodiment the combined therapy may be with monoclonal antibodies blocking immune checkpoint molecules, activating natural killer cells, (CAR) T-cells, tumor-infiltrating lymphocytes or tumor antigen-loaded dendritic cells.

Throughout the description and claims the word "comprise" and variations of the word, are not intended to exclude other technical features, additives, components, or steps. Furthermore, the word "comprise" encompasses the case of "consisting of". Additional objects, advantages and features of the invention will become apparent to those skilled in the art upon examination of the description or may be learned by practice of the invention. The following examples and drawings are provided by way of illustration, and they are not intended to be limiting of the present invention. Reference signs related to drawings and placed in parentheses in a claim, are solely for attempting to increase the intelligibility of the claim, and shall not be construed as limiting the scope of the claim. Furthermore, the present invention covers all possible combinations of particular and preferred embodiments described herein.

### Numbered embodiments

For reasons of completeness, the present invention is also set out in the following numbered embodiments:
1. A combination comprising at least two, at least three, at least four, at least five, or at least six oligonucleotides, wherein said combination reduces or supresses the expression of at least two, at least three, at least four, at least five, or at least six genes selected from the COL5A2, COL11A1, MMP9, ITGB1, TGFB1 and BMP1.
2. The combination of oligonucleotides according to the preceding embodiment, wherein said combination of oligonucleotides reduces or supresses the expression of of the genes COL5A2, COL11A1, MMP9, ITGB1, TGFB1 and BMP1.
3. The combination according to the preceding embodiment, comprising at least six oligonucleotides, each reducing the expression of a different gene selected from the group consisting of COL5A2, COL11A1, MMP9, ITGB1, TGFB1 and BMP1.
4. The combination of oligonucleotides according to any one of the preceding embodiments, wherein the reduction of the expression of the genes is at least 30-99% reduction, in particular the reduction is of at least 60%, or at least 70%, or of at least 80%, or of at least 90%.
5. The oligonucleotide combination according to any one of the preceding embodiments, wherein the oligonucleotide(s) comprise(s) a sequence that is complementary to a target region of a RNA transcript of COL5A2, COL11A1, MMP9, ITGB1, TGFB1 and/or BMP1.
6. The combination of oligonucleotides according to the preceding embodiment, wherein the oligonucleotide(s) is(are) at least 90% complementary to a target region of a RNA transcript of COL5A2, COL11A1, MMP9, ITGB1, TGFB1 and/or BMP1, in particular at least 95% complementary, or at least 98% complementary.
7. The combination of oligonucleotides according to any one of embodiments 5-6, wherein the transcripts are those disclosed in table 11.
8. The combination of oligonucleotides according to any one of embodiments 5-7, wherein the target sequence is comprised within the following regions:
   - for COL5A2 the region delimited by the nucleotides in position 182 to 6410 in NM_000393.5
   - for COL11A1 the region delimited by the nucleotides in position 486 to 5258 in NM_001854.4
   - for MMP9 the region delimited by the nucleotides in position 379 to 2245 in NM_004994.3
   - for ITGB1 the region delimited by the nucleotides in position 185 to 2927 in NM_002211.4
   - for BMP1 the region delimited by the nucleotides in position 547 to 2378 in NM_001199.4
   - for TGFB1 the region delimited by the nucleotides in position 1237 to 2575 in NM_000660.7
9. The combination of oligonucleotides according to, wherein the target sequence:
   - for COL5A2 is within a sequence selected from those disclosed in table 14;
   - for COL11A1 is within a sequence selected from those disclosed in table 15;
   - for MMP9 is within a sequence selected from those disclosed in table 16;
   - for ITGB1 is within a sequence selected from those disclosed in table 17;
   - for BMP1 is within a sequence selected from those disclosed in table 18;
   - for TGFB1 is within a sequence selected from those disclosed in table 19.
10. The combination of oligonucleotides according to any one of the preceding embodiments, wherein the oligonucleotide(s) is(are) 18-25 nucleotides long.
11. The combination of oligonucleotides according to any one of the preceding embodiments, wherein the oligonucleotides are double-stranded.
12. The combination of oligonucleotides according to the preceding embodiment, wherein the oligonucleotides are small interfering RNAs (siRNAs).
13. The combination of oligonucleotides according to the preceding embodiment, wherein the siRNAs are selected from those shown in table 20.
14. The combination of oligonucleotides according to the preceding embodiment, which is an shRNA having a hairpin structure
15. The combination of oligonucleotides according to any one of embodiments 1-10, wherein the oligonucleotides are single-stranded.
16. The combination of oligonucleotides according to the preceding embodiment, wherein the oligonucleotides are selected from micro RNAs (miRNAs), antisense oligonucleotides (AONs).
17. The combination of oligonucleotides according to the preceding embodiment, wherein the oligonucleotides are miRNAs
18. The combination of oligonucleotides according to the preceding embodiment, wherein the miRNAs are selected from hsa-miR-143-3p, hsa-miR-223-3p, hsa-miR-574-5p, hsa-miR-142-3p, hsa-miR-452-5p, hsa-miR-145-5p, hsa-miR-145-3p, hsa-miR-224-5p, hsa-miR-338-5p, hsa-miR-542-3p, hsa-miR-365a-3p, hsa-miR-365b-3p, hsa-miR-335-5p, hsa-miR-30a-5p, hsa-miR-199b-3p, hsa-miR-199a-3p, hsa-miR-223-5p, hsa-miR-152-3p, and hsa-miR-497-5p.
19. The combination of oligonucleotides according to embodiment 16, wherein the oligonucleotides are antisense oligonucleotides comprising DNA and/or RNA
20. The combination of oligonucleotides according to the preceding embodiment, wherein the AONs comprise both DNA and RNA bases.
21. The combination of oligonucleotides according to the preceding embodiment, wherein the AONs are gapmers or altimers.
22. The combination of oligonucleotides according to any one of the preceding embodiments, wherein some or all of the DNA bases have a phosphorothioated backbone.
23. The combination of oligonucleotides according to any one of the preceding embodiments, wherein the oligonucleotides are morpholino oligomers.
24. The combination of oligonucleotides according to any one of the preceding embodiments, wherein some or all of the RNA bases are 2'-0-Methyl (2'0-Me), locked nucleic acid (LNA) bases or 2'-deoxy-2'- fluorobeta-D-arabinonucleic acid (2'FANA) RNA bases.
25. A composition comprising a combination of oligonucleotides according to any one of the preceding embodiments, further comprising heparin or an heparin surrogate.
26. A composition comprising a combination of oligonucleotides according to any one of embodiments 1-24, further comprising a one or more protein inhibitors that bind to the heparin binding domain within the COL5A2, COL11A1, MMP9, ITGB1, TGFB1 and BMP1 proteins.
27. The composition according to any one of embodiments 25-26, that is a pharmaceutical composition comprising a therapeutically effective amount of the oligonucleotide composition and a therapeutically effective amount of heparin, an heparin surrogate, or one or more protein inhibitors that bind to the heparin binding domain within the COL5A2, COL11A1, MMP9, ITGB1, TGFB1 and BMP1 protein, together with pharmaceutically effective excipients and/or carriers.
28. A combination of oligonucleotides as defined in any one of embodiments 1-24, or a composition as defined in any one of embodiments 25-27, for use as a medicament.
28. A combination as defined in any one of embodiments 1-24, or a composition as defined in any one of embodiments 25-27, for use in treating cancer.
29. A combination or composition according to the preceding embodiment, wherein the cancer is a solid tumor.
30. A combination as defined in any one of embodiments 1-24, or a composition as defined in any one of embodiments 25-27, for use in reducing or supressing cancer metastasis.
31. The combination of oligonucleotides or pharmaceutical composition for use according any one of embodiments 28-30, which is for patients having documented resistance to chemotherapy or immunotherapy
32. The combination of oligonucleotides or pharmaceutical composition for use according any one of embodiments 28-31, which is for patients presenting with relapse following cancer treatment
33. The combination of oligonucleotides or pharmaceutical composition for use according to any one of embodiments 28-32, when used in combination with heparin, a heparin surrogate, or one or more protein inhibitors that bind to the heparin binding domain within the COL5A2, COL1 1A1, MMP9, ITGB1, TGFB1 and BMP1 proteins.
34. Heparin, a heparin surrogate, or one or more protein inhibitors that bind to the heparin binding domain within the COL5A2, COL11A1, MMP9, ITGB1, TGFB1 and BMP1 proteins for use in treating cancer, when used in combination with an oligonucleotide, combination of oligonucleotides, or pharmaceutical composition as defined in any one of embodiments 1-24.
35. The oligonucleotide, combination of oligonucleotides, or pharmaceutical composition for use according any one of embodiments 1-24, when used in combination with a surgical intervention, chemotherapy, radiotherapy or immunotherapy

### Examples

### 1. Materials

### 1.1 Design of siRNA oligonucleotides

siRNA sequences were designed using freely available, online tools and then, custom-synthesized by Eurofins Genomics. Scrambled, non-targeting siRNA oligonucleotides served as negative controls.

Online tools used for designing siRNAs:
1. Eurofins Genomics siMAX siRNA Design Tool: https://eurofinsqenomics.eu/en/ecom/tools/sirna-design/
2. GenScript siRNA Target Finder: https://www.genscript.com/tools/sirna-target-finder
3. IDT Custom Dicer-Substrate siRNA (DsiRNA):
   https://www.idtdna.com/site/order/designtool/index/DSIRNA CUSTOM

Software tools to create a scrambled sequence as a negative control:
1. GenScript siRNA Sequence Scrambler: https://www.qenscript.com/tools/create-scrambled-sequence
2. InvivoGen Scramble siRNA: https://www.invivoqen.com/sirnawizard/scrambled.php

Blast via NCBI Blast tool against refseq-RNA database of *Homo sapiens* for both sense and antisense strands to reduce the risk of silencing off-target genes. Blastn program was selected as more suitable for smaller queries. https://blast.ncbi.nlm.nih.qov/Blast.cqi. The queries with >78% coverage with the subject, are considered as a risk factor for off-target effects.

Detailed sequence information of designed siRNA oligonucleotides is provided for each mRNA target in Tables 5-11. The distance of the 1^{st} base of siRNA sequence from the start and the end of the mRNA sequence is also given, showing that the designed siRNA oligos target suitable mRNA sites that cover almost the whole length of the transcript.

**Table 14: siRNA oligonucleotide sequences designed to target proper COL5A2 mRNA sites according to the above-mentioned selection criteria. COL5A2 transcript length is 6829 bases.**

| | **siRNA sequence of sense strand (5'→3')** | **Position of siRNA's target sequence from 5' end of mRNA (bp)** | **GC%** |
|---|---|---|---|
| SEQ ID NO: 67 | AGACCUCUCCUCAUUCUUA | 182-201 | 42.1 |
| SEQ ID NO: 68 | ACACCUGGAGGUGGCAAUA | 452-471 | 52.6 |
| SEQ ID NO: 69 | UCCUGGUUCUCGAGGUGAA | 2653-2672 | 52.6 |
| SEQ ID NO: 70 | ACAAGGAAGUGCUGGAAUC | 3643-3662 | 47.4 |
| SEQ ID NO: 71 | AUACUGGAUUGAUCCUAAC | 4087-4106 | 36.8 |
| SEQ ID NO: 72 | UGAGCACCACCAUCAAUGA | 4681-4700 | 47.4 |
| SEQ ID NO: 73 | UGUGAUGAUUGGAAUUAGA | 5634-5653 | 31.6 |
| SEQ ID NO: 74 | AGACAAGCCAUGCAUACAA | 6139-6158 | 42.1 |
| SEQ ID NO: 75 | AAGGAGAUGUGGUGGUAUA | 6276-6295 | 42.1 |
| SEQ ID NO: 76 | GGCAACAUGACACCAAUUA | 6391-6410 | 42.1 |

**Table 15: siRNA oligonucleotide sequences designed to target proper COL11A1 mRNA sites according to the above-mentioned selection criteria (ORF: Open Reading Frame). COL11A1 transcript length is 7311 bases.**

| | **siRNA sequence of sense strand (5'→3')** | **Position of siRNA's target sequence from 5' end of mRNA (bp)** | **GC%** |
|---|---|---|---|
| *SEQ ID NO: 77* | CACAAUUCUCCAGAGGGAAUAUCAA | 486-511 | 40.0 |
| *SEQ ID NO: 78* | UGAUAGAAGUGAGAGAGCAAUUGUU | 914-939 | 36.0 |
| *SEQ ID NO: 79* | CAUUAUAGUCCAGACUGUGACUCUU | 1056-1081 | 40.0 |
| *SEQ ID NO: 80* | AGAGUAUAAAGAGGCUGAAAGUGUA | 1172-1197 | 36.0 |
| *SEQ ID NO: 81* | CGGAGGCAAACAUCGUUGAUGAUUU | 1237-1262 | 44.0 |
| *SEQ ID NO: 82* | GAUGGAGAUUUAGGCGAAUAUGAUU | 1467-1492 | 36.0 |
| *SEQ ID NO: 83* | CAUCCUGGUUUAAUUGGCCUGAUUG | 4704-4729 | 44.0 |
| *SEQ ID NO: 84* | CAGCCUUUACCAAUCUUGUCCUCCA | 4974-4999 | 48.0 |
| *SEQ ID NO: 85* | CAGACCAAUCCAGCCCGAACUUGUA | 5142-5167 | 52.0 |
| *SEQ ID NO: 86* | GCUCAGGAGAUUCCUUCAAAGUUUA | 5233-5258 | 40.0 |

**Table 16: siRNA oligonucleotide sequences designed to target proper MMP9 mRNA sites according to the above-mentioned selection criteria. MMP9 transcript length is 2336 bases.**

| | **siRNA sequence of sense strand (5'→3')** | **Position of siRNA's target sequence from 5' end of mRNA (bp)** | **GC%** |
|---|---|---|---|
| *SEQ ID NO: 87* | CAUCACCUAUUGGAUCCAA | 379-398 | 42.1 |
| *SEQ ID NO: 88* | ACUACUCGGAAGACUUGCC | 399-418 | 52.6 |
| *SEQ ID NO: 89* | AGGAGUACUCGACCUGUAC | 1086-1105 | 52.6 |
| *SEQ ID NO: 90* | UGCCUGCAACGUGAACAUC | 1561-1580 | 52.6 |
| *SEQ ID NO: 91* | ACGUGAACAUCUUCGACGC | 1569-1588 | 52.6 |
| *SEQ ID NO: 92* | GUACUGGCGAUUCUCUGAG | 1633-1652 | 52.6 |
| *SEQ ID NO: 93* | ACUGGUAUUCUGUUCUGGA | 2225-2244 | 42.1 |
| *SEQ ID NO: 94* | CUGGUAUUCUGUUCUGGAG | 2226-2245 | 47.4 |

**Table 17: siRNA oligonucleotide sequences designed to target proper ITGB1 mRNA sites according to the above-mentioned selection criteria. ITGB1 transcript length is 3735 bases.**

| | **siRNA sequence of sense strand (5'→3')** | **Position of siRNA's target sequence from 5' end of mRNA (bp)** | **GC%** |
|---|---|---|---|
| SEQ ID NO: 95 | AUCAUGUGGAGAAUGUAUA | 185-204 | 31.6 |
| SEQ ID NO: 96 | UCAUGUGGAGAAUGUAUAC | 186-205 | 36.8 |
| SEQ ID NO: 97 | AAGACUGUGAUGCCUUACA | 654-673 | 42.1 |
| SEQ ID NO: 98 | AAUGUGCUCAGUCUUACUA | 747-766 | 36.8 |
| SEQ ID NO: 99 | AUGUGCUCAGUCUUACUAA | 748-767 | 36.8 |
| SEQ ID NO: 100 | UGUACACAAUGAGCCAUUA | 1006-1025 | 36.8 |
| SEQ ID NO: 101 | CAAUGAGCCAUUAUUAUGA | 1012-1031 | 31.6 |
| SEQ ID NO: 102 | GUCAGCAGUAGGAACAUUA | 1148-1167 | 42.1 |
| SEQ ID NO: 103 | AUUGUCAGAAGGCGUAACA | 1250-1269 | 42.1 |
| SEQ ID NO: 104 | AUGUCCUGCUAGCUAGUUA | 2908-2927 | 42.1 |

**Table 18: siRNA oligonucleotide sequences designed to target proper TGFB1 mRNA sites according to the above-mentioned selection criteria. TGFB1 transcript length is 2780 bases.**

| | **siRNA sequence of sense strand (5'→3')** | **Position of siRNA's target sequence from 5' end of mRNA (bp)** | **GC%** |
|---|---|---|---|
| SEQ ID NO: 105 | UCUAUGACAAGUUCAAGCA | 1237-1256 | 36.8 |
| SEQ ID NO: 106 | GCAGAGUACACACAGCAUA | 1253-1272 | 47.4 |
| SEQ ID NO: 107 | CACAUCAGAGCUCCGAGAA | 1286-1305 | 52.6 |
| SEQ ID NO: 108 | CACACUGCAAGUGGACAUC | 1568-1587 | 52.6 |
| SEQ ID NO: 109 | GCAGCUGUCCAACAUGAUC | 2009-2028 | 52.6 |
| SEQ ID NO: 110 | ACACUACUGUAGUUAGAUC | 2339-2358 | 36.8 |
| SEQ ID NO: 111 | GUGCCUUACAUUAAUGAAC | 2388-2407 | 36.8 |
| SEQ ID NO: 112 | UGAACUCAUUCAGUCACCA | 2402-2421 | 42.1 |
| SEQ ID NO: 113 | CUCAUUCAGUCACCAUAGC | 2406-2425 | 47.4 |
| SEQ ID NO: 114 | AGAUAGUAGUUCAGGCCAG | 2556-2575 | 47.4 |

**Table 19: siRNA oligonucleotide sequences designed to target proper BMP1 mRNA sites according to the above-mentioned selection criteria. BMP1 transcript length is 2506 bases.**

| | **siRNA sequence of sense strand (5'→3')** | **Position of siRNA's target sequence from 5' end of mRNA (bp)** | **GC%** |
|---|---|---|---|
| SEQ ID NO: 115 | CGAGGACAGCUAUAUUGUG | 547-566 | 47.4 |
| SEQ ID NO: 116 | GGACAGCUAUAUUGUGUUC | 550-569 | 42.1 |
| SEQ ID NO: 117 | AGACAGCACAGGCAACUUC | 1015-1034 | 52.6 |
| SEQ ID NO: 118 | UGGCUACUCUGCUCACAUG | 1054-1073 | 52.6 |
| SEQ ID NO: 119 | GAAGAUCAUCCUGAACUUC | 1108-1127 | 42.1 |
| SEQ ID NO: 120 | AGAUCAUCCUGAACUUCAC | 1110-1129 | 42.1 |
| SEQ ID NO: 121 | AGGACUAUGGCCACAUUCA | 1356-1375 | 47.4 |
| SEQ ID NO: 122 | GGACUAUGGCCACAUUCAA | 1357-1376 | 47.4 |
| SEQ ID NO: 123 | GUUCUGUGGUUCUGAGAAG | 2026-2045 | 47.4 |
| SEQ ID NO: 124 | GAACUGGUGCUCUCUUCUC | 2359-2378 | 52.6 |

**Table 20: List of the siRNA oligonucleotides selected for further experimental assays. Only sense strands are shown. The guide (antisense) strand of siRNAs contained a 2-nucleotide asymmetric 3'-overhang in the was composed of 2'-deoxythymidine (dTdT). The dTdT overhang confers increased nuclease resistance of siRNAs in the cell culture medium and within transfected cells.**

| **Gene** | **Targeted Transcripts** | **siRNA oligonucleotide sequence** |
|---|---|---|
| ***COL5A2*** | NM_000393.5 | Sense strand (5'-3'): AGACCUCUCCUCAUUCUUA (SEQ ID NO: 125) |
| | | |
| ***COL11A1*** | NM_001854.4 | Sense strand (5'-3'): GCUCAGGAGAUUCCUUCAAAGUUUA (SEQ ID NO: 126) |
| | NM_080629.3 | |
| | NM_080630.4 | |
| | NM_001190709.2 | |
| ***MMP9*** | NM_004994.3 | Sense strand (5'-3'): CAUCACCUAUUGGAUCCAA (SEQ ID NO: 127) |
| | | |
| ***ITGB1*** | NM_002211.4NM_033668.2 NM_133376.3 | Sense strand (5'-3'): GUCAGCAGUAGGAACAUUA (SEQ ID NO: 128) |
| | | |
| ***BMP1*** | NM_001199.4 | Sense strand (5'-3'): GGACUAUGGCCACAUUCAA (SEQ ID NO: 129) |
| | NM_006129.5 | |
| ***TGFB1*** | NM_000660.7 | Sense strand (5'-3'): GCAGAGUACACACAGCAUA (SEQ ID NO: 130) |
| | | |
| **Negative Control** | Non-targeting | Sense strand (5'-3'): GCCUAUCUUACACUCCUAU (SEQ ID NO: 131) |
| | | |
| **Negative Control** | Non-targeting | Sense strand (5'-3'): GCGUCUUAAGGAACUAGUCUAUUCA (SEQ ID NO: 132) |
| | | |

### 1.2 Cell cultures

The human osteosarcoma mesenchymal cell line U-2 OS (Ref Number: HTB-96^{™}) and the human lung adenocarcinoma A549 (Ref Number: CCL-185) cell line were obtained from the American Type Culture Collection (ATCC, USA). The cells were grown in DMEM supplemented with 10% FBS, 100 U/mL penicillin, and 100 µg/mL streptomycin under a humidified atmosphere with 5% CO₂ at 37 °C.

### 1.3 siRNA transfection

Confluent cells were seeded at a density of 50,000 cells/well in 24-well plates. Cells were maintained in antibiotic-free DMEM supplemented with 10% FBS, for 24 hours and then, were transfected with one of the gene-specific siRNAs (10 nM) or the negative, non-targeting control (10 nM), using Lipofectamine RNAi MAX (Invitrogen), according to the manufacturer's instructions. Cells were incubated for 24 and 48 hours and were, then, subjected to further analysis. Each condition was tested in duplicate transfections.

### 1.4 RNA extraction and RT-qPCR

Total RNA was extracted from transfected and control cells after 24 and 48 hours of incubation, using Nucleospin RNA kit (Macherey Nagel GmbH & Co. KG, Düren, Germany). RNA concentration and quality were determined by measurement of the samples in the NanoDrop One^{c} (Thermo Scientific). One ug of RNA was reverse transcribed to cDNA using High-Capacity cDNA Reverse Transcription Kit (Applied Biosystems), as recommended by the manufacturer. RT-qPCR was performed using Maxima SYBR Green/ROX qPCR Master Mix (Thermo Scientific) on the Rotor-Gene Q apparatus (Qiagen). Cycling conditions were as follows: pre-activation step at 95 °C for 10 min, 40 cycles of denaturation at 95°C for 15 s, annealing at 60°C for 30 s and elongation at 72°C for 30 s and a final melting curve analysis step. All samples were run in duplicates. Heat *Shock Protein Family A (Hsp70) Member* 8 *(HSPA8)* was selected as a reference gene for the normalization of gene expression levels. Gene knockdown was calculated by the 2^{-ΔΔCt} method, using non-transfected cells as the reference sample. Primer sequences (Table 12) were designed using the NCBI Primer-Blast designing tool (BMC Bioinformatics, 2012;13:134. Primer-BLAST: a tool to design target-specific primers for polymerase chain reaction. Ye J. et al.).

**Table 21: List of RT-qPCR primers used for the evaluation of siRNA silencing efficiency.**

| **Gene** | | **Primer sequence (5'-3')** | **Amplicon size (bp)** |
|---|---|---|---|
| ***HSPA8*** | F: | GGCTTCCTTCGTTATTGGAGC (SEQ ID NO: 133) | 198 |
| | R: | ACCGATCAACCGTTCAGTGT (SEQ ID NO: 134) | |
| ***COL5A2*** | F: | ACTGCAACATGGAAACAGGAGA (SEQ ID NO: 135) | 157 |
| | R: | GATTGGTGGTCTCCATAAGCG (SEQ ID NO: 136) | |
| ***COL11A1*** | F: | ACACTGTATGATGGTTGTGCG (SEQ ID NO: 137) | 152 |
| | R: | ACAGGACCAACTTCAAATCCGA (SEQ ID NO: 138) | |
| ***ITGB1*** | F: | AAGCGAAGGCATCCCTGAAA (SEQ ID NO: 139) | 199 |
| | R: | GCAGACGCACTCTCCATTGT (SEQ ID NO: 140) | |
| ***MMP9*** | F: | CCTCTGGAGGTTCGACGTG (SEQ ID NO: 141) | 158 |
| | R: | AACTCACGCGCCAGTAGAAG (SEQ ID NO: 142) | |
| ***TGFB1*** | F: | GCAACAATTCCTGGCGATACC (SEQ ID NO: 143) | 198 |
| | R: | TAGTGAACCCGTTGATGTCCA (SEQ ID NO: 144) | |
| ***BMP1*** | F: | GCCTGTGCTGGTACGACTAT (SEQ ID NO: 145) | 189 |
| | R: | CGCAGATGGCTTCGTAGACT (SEQ ID NO: 146) | |

### 1.4. miRNA sequencing

Total RNA was extracted from plasma samples and miRNAs were detected with Next Generation Sequencing techniques. Comparisons between cancer patients and healthy individuals were performed with counts per million in the samples.

### 1.5. Western blot analysis

Cell culture experiments were performed in 6-well plates with 2 × 10⁶ cells/well. Following overnight starvation in serum-free culture medium, cells were incubated with or without 10 nM of human tissue-type plasminogen activator (tPA, Sigma-Aldrich), for 48 h to induce MMP9 protein secretion. For COL11A1 experiments, stimulation of cells with tPA was omitted. Afterwards, cells were treated with or without 50 international units (IU) of low molecular weight heparin (LMWH) per well, in the absence or presence of 10 nM siRNA oligos for 24 h. The effect of four different types of LMWH on MMP9 and COL11A1 expression levels was analyzed: Clexane - enoxaparin sodium; Sanofi-Aventis, Fragmin - dalteparin sodium; Pfizer, INNOHEP- tinzaparin sodium; Celgene Corporation and Fraxiparine - Nadroparin calcium; Sanofi.

Cell supernatant was collected for the analysis of secreted protein levels 24 h post-treatment with heparin and/or siRNA. Supernatants were processed with Amicon Ultra-15 Centrifugal Filter Unit with Ultracel-3 membrane (Merk Millipore) for concentration of proteins with a molecular mass greater than 3 kDa, according to the manufacturer's instructions. Equal protein amounts were resolved on 4-12% w/v Bis-Tris acrylamide gels followed by western blot analysis onto polyvinylidene difluoride membranes (Macherey Nagel). Membranes were blocked with 5% w/v bovine albumin (BSA) in PBS-Tween 0.1% v/v at room temperature for 1 h. Then, membranes were incubated overnight at 4°C with the primary recombinant anti-MMP9 antibody (1:2000 in 5% w/v BSA in PBS-Tween 0.1% v/v, product code: ab76003, Abcam) and recombinant anti-GAPDH antibody (1:2000 in 5% w/v BSA in PBS-Tween 0.1% v/v, product code: ab181602, Abcam) as a loading control. Membranes were washed with PBS-Tween 0.1% v/v and incubated for 1 h at room temperature with goat anti-rabbit IgG H&L conjugated with horseradish peroxidase (1:10000 in 5% w/v BSA in PBS-Tween 0.1% v/v, product code: ab97051, Abcam), and the immunoblots were detected by the high sensitivity, enhanced chemiluminescence (ECL) substrate kit (Abcam) and X-Ray film (Kisker Biotech). The density of the bands appeared on the X-ray film during the detection was quantified using the imaged (FIJI) software.

### 1.6. In vitro cell migration assay

Cells were grown to confluency in 24-well plates and maintained in antibiotic-free DMEM supplemented with 10% FBS for 24 hours. A wound was created by scratching the cell monolayer in a straight line with a sterile P-200 pipette tip. Cells were then washed twice with PBS and treated with 100 international units (IU)/mL of low molecular weight heparin (LMWH), in the absence or presence of 10 nM siRNA oligos targeting *COL11A1* or *MMP9* mRNA. Cells were further cultured in antibiotic-free medium. Scratch re-population was recorded by microscopy images of wound closure at 0, 12 and 24 hrs. Quantification of the wound area invaded during 24 h by untreated and treated cells was determined in relative units, compared to the initial wound area (t = 0 h) for each condition.

### 1.7. Statistical analysis

Data are expressed as mean ± standard error (SEM) of the mean of two independent experiments. Two-way ANOVA with Bonferroni post-hoc analysis was used to assess statistical significance. A value of p < 0.05 was considered as statistically significant.

### 2. RESULTS

### 2.1 Extracellular matrix - related molecules are highly expressed in U-2 OS and A549 carcinogenic cell lines

U-2 OS (Ref Number: HTB-96^{™}) and A549 (Ref Number: CCL-185) cell lines were obtained from the American Type Culture Collection (ATCC, USA).

We verified that U-2 OS cells show high levels of *COL5A2, COL11A1, MMP9, ITGB1, BMP1* and *TGFB1* transcription, which are known to be implicated in ECM remodeling. As shown in Figure 1A-F, which depicts the amplification curves derived from RT-qPCR experiments, the cycle threshold (Ct) values of target-genes in non-transfected U-2 OS cells are ≤ 20, indicating high, endogenous expression levels. In siRNA-transfected U-2 OS cells (see Section 1.3), the amplification curve of each target-gene has shifted to the right leading to increased Ct value, whereas the Ct values of HSPA8 remain almost unaffected between non-transfected and transfected cells (Figure 1A-F).

Similar high transcription levels of ECM constituents, cell-ECM adhesion molecules and metalloproteinases were observed in A549 cells, as well (data not shown).

### 2.2 Differential expression of cell-free miRNAs targeting ECM - related genes was identified in cancer patients

The expression profile of cell-free miRNAs in plasma of cancer patients compared to healthy individuals, was determined through small-RNA sequencing analysis. Thirty-six miRNAs were identified to be differentially expressed in cancer patients. Among them, 15 were upregulated, while 21 were downregulated.

The experimentally validated microRNA-target interactions database (miRTarBase) and the prediction tool of miRNA targets (TargetScan 8.0) were used to assess the genes targeted by the DE miRNAs. Our analysis focused on genes/proteins involved in the ECM structure, degradation and cellular interaction. Most of the miRNAs targeting the above-mentioned genes were shown to be downregulated in cancer patients, leading to potentially increased gene and protein expression levels. These data are shown in Table 11 and Figures 2 & 3.

**Table 22: Differentially expressed miRNAs in cancer and in silico predicted target-genes.**

| *miRNA* | *miRBase Accession number (Release 22*.*1*) | *Up or Down-regulated in cancer* | *Fold change* | *Predicted target-genes* | *miRTarBase* | *TargetScan* |
|---|---|---|---|---|---|---|
| hsa-miR-144-3p | MIMAT0000436 | ↑ | 1.49 | TGFB1, COL5A2, COL11A1, ITGA6 | Yes | Yes |
| hsa-miR-143-3p | MIMAT0000435 | ↓ | 0.56 | COL5A1, COL5A2, COL11A1, ITGA6 | Yes | Yes |
| hsa-miR-15a-5p | MIMAT0000068 | ↑ | 1.47 | ITGB1 | No | Yes |
| hsa-miR-223-3p | MIMAT0000280 | ↓ | 0.44 | ITGB1 | Yes | Yes |
| hsa-miR-16-2-3p | MIMAT0004518 | ↑ | 1.51 | COL11A1, COL5A2, ITGA4, ITGA6, ITGB1 | No | Yes |
| hsa-miR-92a-3p | MIMAT0000092 | ↑ | 1.26 | COL5A1, ITGA6 | No | Yes |
| hsa-miR-574-5p | MIMAT0004795 | ↓ | 0.63 | COL5A1, ITGA6 | Yes | Yes |
| hsa-miR-101-3p | MIMAT0000099 | ↑ | 1.27 | ITGA3 | No | Yes |
| hsa-miR-25-3p | MIMAT0000081 | ↑ | 1.27 | COL5A1, ITGA6, ITGB1 | Yes | Yes |
| hsa-miR-4508 | MIMAT0019045 | ↑ | 2.64 | ITGA4 | No | Yes |
| hsa-miR-1290 | MIMAT0005880 | ↑ | 11.31 | COL5A1 | No | Yes |
| hsa-miR-7704 | MIMAT0030019 | ↑ | 3.73 | COL5A1, ITGA3, BMP1 | No | Yes |
| hsa-miR-142-3p | MIMAT0000434 | ↓ | 0.54 | ITGA4 | No | Yes |
| hsa-miR-10396b-5p | MIMAT0041635 | ↑ | 6.96 | - | | |
| hsa-miR-10396a-5p | MIMAT0041623 | ↑ | 6.96 | - | | |
| hsa-miR-452-5p | MIMAT0001635 | ↓ | 0.33 | MMP2 | Yes | No |
| hsa-miR-145-5p | MIMAT0000437 | ↓ | 0.47 | COL5A1 | Yes | No |
| hsa-miR-184 | MIMAT0000454 | ↓ | 0.18 | - | | |
| hsa-miR-145-3p | MIMAT0004601 | ↓ | 0.5 | ITGA3 | No | Yes |
| hsa-miR-224-5p | MIMAT0000281 | ↓ | 0.38 | ITGA6 | No | Yes |
| hsa-miR-338-5p | MIMAT0004701 | ↓ | 0.38 | COL5A1, COL5A2 | Yes | Yes |
| hsa-miR-542-3p | MIMAT0003389 | ↓ | 0.57 | COL5A1, ITGA3, ITGB1 | No | Yes |
| hsa-let-7b-5p | MIMAT0000063 | ↑ | 1.80 | ITGA3, COL5A2, COL11A1 | Yes | Yes |
| hsa-miR-33b-5p | MIMAT0003301 | ↑ | 1.62 | COL5A1, COL5A2, ITGB1 | No | Yes |
| hsa-miR-365a-3p | MIMAT0000710 | ↓ | 0.53 | ITGA6, BMP1 | No | Yes |
| hsa-miR-365b-3p | MIMAT0022834 | ↓ | 0.53 | ITGA6, BMP1 | No | Yes |
| hsa-miR-1246 | MIMAT0005898 | ↑ | 4.92 | ITGB1 | No | Yes |
| hsa-miR-532-3p | MIMAT0004780 | ↑ | 1.44 | COL5A1, ITGA3, ITGB1, TGFB1 | No | Yes |
| hsa-miR-335-5p | MIMAT0000765 | ↓ | 0.59 | COL11A1, MMP2, BMP1 | Yes | No |
| hsa-miR-30a-5p | MIMAT0000087 | ↓ | 0.58 | ITGA4, ITGA6 | Yes | Yes |
| hsa-miR-199b-3p | MIMAT0004563 | ↓ | 0.56 | MMP2, ITGA3, ITGA6 | Yes | Yes |
| hsa-miR-199a-3p | MIMAT0000232 | ↓ | 0.56 | MMP2, ITGA3, ITGA6 | Yes | Yes |
| hsa-miR-223-5p | MIMAT0004570 | ↓ | 0.47 | ITGA4 | No | Yes |
| hsa-miR-152-3p | MIMAT0000438 | ↓ | 0.54 | ITGA3 | No | Yes |
| hsa-miR-24-2-5p | MIMAT0004497 | ↓ | 0.56 | - | | |
| hsa-miR-497-5p | MIMAT0002820 | ↓ | 0.57 | ITGB1 | No | Yes |

### 2.3 siRNA-mediated knockdown of target-genes in U-2 OS and A549 cell lines

Based on the above data, we designed gene-specific, synthetic, small interfering RNA (siRNA) oligonucleotides to induce gene silencing of *COL5A2, COL11A1, MMP9, ITGB1, TGFB1* and *BMP1* in osteosarcoma U-2 OS and lung adenocarcinoma A549 cell lines, to explore their therapeutic potential in controlling invasion and metastasis in cancer.

The knockdown efficiency in U-2 OS cells after 1 and 2 days of siRNA transfection, was determined by RT-qPCR. Silencing of all target-genes was successfully achieved, as shown by the significantly decreased mRNA expression levels in the siRNA-transfected groups compared to non-transfected U-2 OS cells (Figure 2). The obtained reduction in relative mRNA expression ranged from approximately 79% in the case of COL5A2 (Figure 2A) to 95% for *TGFB1* (Figure 2F). Similar results were obtained from siRNA transfection experiments in A549 cells (data not shown).

### 2.4. Co-treatment with siRNA and heparin suppresses MMP9 and COL11A1 secretion leading to decreased migration ability

Data shown in Figures 3 and 4 highlight that administration of low molecular weight heparin (LMWH), along with siRNAs treatment, further suppresses MMP9 and COL11A1 levels, inhibiting migratory behavior of cancer cells (Figure 5).

### Citation List

Karlin and Altschul (1990) Proc. Natl. Acad. Sci. USA 87:2264-68
Karlin and Altschul (1993) Proc. Natl. Acad. Sci. USA 90:5873-77
Altschul, et al. (1990) J. Mol. Biol. 215:403-10
Altschul et al, (1997) Nucleic Acids Res. 25(17):3389-3402.
Konermann et al (2014) Genome-scale transcriptional activation by an engineered CRISPR-Cas9 complex, Nature volume 517, pages 583-588 (2015)
Eurofins Genomics siMAX siRNA Design Tool: https://eurofinsgenomics.eu/en/ecom/tools/sirna-design/
GenScript siRNA Target Finder: https://www.genscript.com/tools/sirna-target-finder
IDT Custom Dicer-Substrate siRNA (DsiRNA): https://www.idtdna.com/site/order/designtool/index/DSIRNA_CUSTOM
GenScript siRNA Sequence Scrambler: https://www.genscript.com/tools/create-scrambled-sequence InvivoGen Scramble siRNA: https://www.invivoqen.com/sirnawizard/scrambled.php
NCBI Blast tool: https://blast.ncbi.nlm.nih.gov/Blast.cqi.
NCBI Primer-Blast designing tool (BMC Bioinformatics, 2012;13:134. Primer-BLAST: a tool to design target-specific primers for polymerase chain reaction. Ye J. et al.).

## Claims

1. A combination of oligonucleotides, wherein said combination reduces the expression of the genes COL5A2, COL11A1, MMP9, ITGB1, TGFB1 and BMP1.

2. The combination according to claim 1, wherein said combination reduces de expression of the genes in at least 60-99%.

3. The combination according to any one of claims 1-2-, comprising at least six oligonucleotides, each reducing the expression of a different gene selected from the group consisting of COL5A2, COL11A1, MMP9, ITGB1, TGFB1 and BMP1.

4. The combination according to claim 3, wherein the oligonucleotides comprise a sequence that is complementary to a target sequence of a RNA transcript of COL5A2, COL11A1, MMP9, ITGB1, TGFB1 or BMP1.

5. The combination of oligonucleotides according to claim 4, wherein the transcripts are those disclosed in table 11.

6. The combination of oligonucleotides according to any one of claims 4-5, wherein the target sequence is comprised within the following regions:
- for COL5A2 the sequence delimited by the nucleotides in position 182 to 6410 in NM_000393.5 (SEQ ID NO: 1);
- for COL11A1 the sequence delimited by the nucleotides in position 486 to 5258 in NM_001854.4 (SEQ ID NO: 2);
- for MMP9 the sequence delimited by the nucleotides in position 379 to 2245 in NM_004994.3 (SEQ ID NO: 3);
- for ITGB1 the sequence delimited by the nucleotides in position 185 to 2927 in NM_002211.4 (SEQ ID NO: 4);
- for BMP1 the sequence delimited by the nucleotides in position 547 to 2378 in NM_001199.4 (SEQ ID NO: 5);
- for TGFB1 the sequence delimited by the nucleotides in position 1237 to 2575 in NM_000660.7 (SEQ ID NO: 6).

7. The combination of oligonucleotides according to claim 6, wherein the target sequence:
- for COL5A2 is within a sequence selected from those disclosed in table 14;
- for COL11A1 is within a sequence selected from those disclosed in table 15;
- for MMP9 is within a sequence selected from those disclosed in table 16;
- for ITGB1 is within a sequence selected from those disclosed in table 17;
- for BMP1 is within a sequence selected from those disclosed in table 18;
- for TGFB1 is within a sequence selected from those disclosed in table 19.

8. The combination of oligonucleotides according to any one of claims 1-7, wherein the oligonucleotides are 18-25 nucleotides long.

9. The combination of oligonucleotides according to any one of claims 1-8, wherein the oligonucleotides are selected from small interfering RNAs (siRNAs), micro RNAs (miRNAs), and antisense oligonucleotides (AONs).

10. The combination of oligonucleotides according to the preceding claim, wherein the oligonucleotides are siRNAs selected from those shown in table 20 or miRNAs selected from hsa-miR-143-3p, hsa-miR-223-3p, hsa-miR-574-5p, hsa-miR-142-3p, hsa-miR-452-5p, hsa-miR-145-5p, hsa-miR-145-3p, hsa-miR-224-5p, hsa-miR-338-5p, hsa-miR-542-3p, hsa-miR-365a-3p, hsa-miR-365b-3p, hsa-miR-335-5p, hsa-miR-30a-5p, hsa-miR-199b-3p, hsa-miR-199a-3p, hsa-miR-223-5p, hsa-miR-152-3p, and hsa-miR-497-5p.

11. A composition comprising a combination of oligonucleotides according to any one of claims 1-10, optionally further comprising heparin, a heparin surrogate, or one or more protein inhibitors that bind to the heparin binding domain within the COL5A2, COL11A1, MMP9, ITGB1, TGFB1 and/or BMP1 proteins.

12. The composition according to claim 11, that is a pharmaceutical composition comprising a therapeutically effective amount of the oligonucleotide composition and, optionally, a therapeutically effective amount of heparin, an heparin surrogate, or one or more protein inhibitors that bind to the heparin binding domain within the COL5A2, COL11A1, MMP9, ITGB1, TGFB1 and/or BMP1 proteins, together with pharmaceutically effective excipients and/or carriers.

13. A combination as defined in any one of claims 1-10, or a composition as defined in any one of claims 11-12, for use as a medicament.

14. A combination as defined in any one of claims 1-10, or a composition as defined in any one of claims 11-12, for use in treating cancer, in particular, for use in reducing or supressing cancer metastasis.

15. A combination of oligonucleotides as defined in any one of claims 1-10, for use in treating cancer, when used in combined therapy with heparin, an heparin surrogate, or one or more protein inhibitors that bind to the heparin binding domain within the COL5A2, COL11A1, MMP9, ITGB1, TGFB1 and BMP1 proteins or, alternatively, Heparin, an heparin surrogate, or one or more protein inhibitors that bind to the heparin binding domain within the COL5A2, COL11A1, MMP9, ITGB1, TGFB1 and BMP1 proteins for use in treating cancer, when used in combined therapy with a combination of oligonucleotides as defined in any one of claims 1-10.
